# EUROPEAN PATENT SPECIFICATION

(11) **EP 2 550 039 B1**
(45) Date of publication and mention of the grant of the patent: **04.06.2014**
(21) Application number: 11710650.0
(22) Date of filing: 21.03.2011
(51) Int. Cl.: A61M 5/142, F04C 5/00, F04C 11/00, F04C 2/356, F04C 14/06

(54) **ROTARY INFUSION PUMPS**
DREHINFUSIONSPUMPEN
POMPE À PERFUSION ROTATIVE

(30) Priority: 23.03.2010 US 316612 P
(43) Date of publication of application: 30.01.2013
(73) Proprietor: Baxter International Inc., Deerfield, Illinois 60015 (US); Baxter Healthcare SA, 8152 Glattpark (Opfikon) (CH)
(72) Inventor: DELCASTILLO, Jorge A., Des Plaines, Illinois 60016 (US); LIN, Rongsheng, Buffalo Grove, Illinois 60089 (US); ZHU, Hong, Buffalo Grove, Illinois 60089 (US); DUDAR, Thomas E., Palatine, Illinois 60067 (US)
(74) Representative: Potter Clarkson LLP
(86) International application number: PCT/US2011/029145
(87) International publication number: WO 2011/119464

(56) References cited:
- WO-A1-2005/010367
- US-B2- 7 674 100

## Description

### BACKGROUND

Medical patients sometimes require delivery of either continuous medication or medication at set periodic intervals. Drug infusion pumps have been developed to provide controlled drug infusion, wherein the drug can be administered at a set rate that maintains the drug concentration within a therapeutic margin and out of an unnecessary or possibly toxic range. Drug infusion pumps deliver drugs to the patient at a controlled rate and typically do not require frequent attention.

Drug infusion pumps have been developed to deliver intravenous ("IV") therapy to patients both in and outside of a clinical setting. Outside a clinical setting, doctors have found that in many instances patients can return to substantially normal lives, provided that they receive periodic or continuous IV administration of medication. Among the types of therapies requiring this kind of administration are antibiotic therapy, chemotherapy, pain control therapy, nutritional therapy, and several other types known by those skilled in the art. In many cases, patients receive multiple daily therapies. Certain medical conditions require infusions of drugs in solution over relatively short periods, such as from thirty minutes to two hours. These conditions and others have combined to promote the development of increasingly lightweight, portable or ambulatory infusion pumps that can be worn by a patient and are capable of administering a continuous supply of medication at a desired rate, or provide several doses of medications at scheduled intervals.

Infusion pumps include elastomeric pumps, which squeeze solution from flexible containers, such as balloons, into IV tubing for delivery to the patient. Alternatively, spring-loaded pumps pressurize the solution containers or reservoirs. Certain pump designs use cartridges containing flexible compartments that are squeezed by pressure rollers for discharging the solutions. Other infusion pumps squeeze pre-filled single dosage IV bags using a roller. Certain known infusion pumps use syringes and a drive mechanism that moves a plunger of the syringe to deliver fluid to a patient. Typical syringe infusion pumps include a housing adapted to receive a syringe assembly, a drive mechanism adapted to move the syringe plunger, a pump control unit having a variety of operating controls, and a power source for powering the pump including the drive mechanism and controls.

While the above-discussed prior art and other designs have recognized the need for an infusion pump that is smaller and more compact for mobile use, each has failed to adequately address the ambulatory pump market's price sensitivity due to pressure from outside competition and reimbursement constraints, especially in emerging countries.

A need accordingly exists for a portable, ambulatory type infusion pump, which is cost effective and meets operability requirements.

### SUMMARY

Aspects of the subject matter described herein may be useful alone or in combination one or more other aspect described herein. Without limiting the following description, in a first aspect of the present disclosure, an infusion pump includes a housing; a rotor inserted into the housing, the rotor including a first ring of surfaces that form channels with the housing, and a second ring of surfaces that form channels with the housing, the second channel forming surfaces radially offset from the first channel forming surfaces; and a motor operably engaged to rotate the rotor to cause the first and second channel forming surfaces to pump a flow of fluid, the radial offsetting of the second channel forming surfaces from the first channel forming surfaces dampening a pulsatility of the flow of fluid. WO2005/010367 describes a similar pump with a rotor and two offset rings of surfaces for dampening a pulsatility of the flow of fluid delivered by the pump.

In a second aspect of the present disclosure, which may be used in combination with the first aspect, the first channel forming surfaces are sized to produce a first flowrate, and the second channel forming surfaces are sized to produce a second flowrate different than the first flowrate.

In a third aspect of the present disclosure, which may be used in combination with the second aspect, the rotor includes a third ring of surfaces that form channels with the housing, the third channel forming surfaces sized to produce a third flowrate different than the first and second flowrates.

In a fourth aspect of the present disclosure, which may be used in combination with any one or more of the preceding aspects, the rotor includes a third ring of surfaces that form channels with the housing, the third channel forming surfaces radially offset from the first and second channel forming surfaces to further dampen the pulsatility of the flow of fluid.

In a fifth aspect of the present disclosure, which may be used in combination with any one or more of the preceding aspects, a selector is provided and configured to slide linearly to selectively (i) occlude flow to both the first ring of channel forming surfaces and the second ring of channel forming surfaces, (ii) occlude flow to the second ring of channel forming surfaces only, or (iii) allow flow to both the first ring of channel forming surfaces and the second ring of channel forming surfaces.

In a sixth aspect of the present disclosure, which may be used in combination with any one or more of the preceding aspects, a selector is provided and configured to slide linearly to selectively (i) close a first membrane valve for the first ring of channel forming surfaces and a second membrane valve for the second ring of channel forming surfaces, (ii) close the second membrane valve for the second ring of channel forming surfaces only, or (iii) allow both the first and second membrane valves to be open.

In a seventh aspect of the present disclosure, which may be used in combination with any one or more of the preceding aspects, a selector is provided and configured to rotate to selectively (i) occlude flow to both the first ring of channel forming surfaces and the second ring of channel forming surfaces, (ii) occlude flow to the second ring of channel forming surfaces only, or (iii) allow flow to both the first ring of channel forming surfaces and the second ring of channel forming surfaces.

In an eighth aspect of the present disclosure, which may be used in combination with any one or more of the preceding aspects, a selector is provided and configured to rotate to selectively (i) close a first tube for the first ring of channel forming surfaces and a second tube for the second ring of channel forming surfaces, (ii) close the second tube for the second ring of channel forming surfaces only, or (iii) allow both the first and second tubes to be open.

In a ninth aspect of the present disclosure, which may be used in combination with any one or more of the preceding aspects, a selector is provided and configured to rotate to selectively (i) close a first tube for both the first ring of channel forming surfaces and the second ring of channel forming surfaces, (ii) close a second tube for the second ring of channel forming surfaces only, or (iii) allow both the first and second tubes to be open.

In a tenth aspect of the present disclosure, which may be used in combination with any one or more of the preceding aspects, a selector is provided and configured to rotate to selectively (i) close a first membrane valve for the first ring of channel forming surfaces and a second membrane valve for the second ring of channel forming surfaces, (ii) close the second membrane valve for the second ring of channel forming surfaces only, or (iii) allow both the first and second membrane valves to be open.

In an eleventh aspect of the present disclosure, which may be used in combination with any one or more of the preceding aspects, the housing and rotor are part of a disposable portion of the infusion pump.

In a twelfth aspect of the present disclosure, which may be used in combination with any one or more of the preceding aspects, the housing is connected to at least one of: a supply bag line, a patient line and a supply bag.

In a thirteenth aspect of the present disclosure, which may be used in combination with any one or more of the preceding aspects, at least one of the motor and the housing is part of a reusable portion of the infusion pump.

In a fourteenth aspect of the present disclosure, which may be used in combination with any one or more of the preceding aspects, the housing and the rotor are part of a first portion of the infusion pump and an occluding selector and the motor are part of a second portion of the infusion pump, the first and second portions being configured to be connected such that (i) the housing comes into operational communication with the selector and (ii) the rotor comes into operational communication with the motor.

In a fifteenth aspect of the present disclosure, which may be used in combination with the fourteenth aspect, the first and second portions are configured to be connected such that that (i) the housing coming into operational communication with the selector and (ii) the rotor coming into operational communication with the motor occur virtually simultaneously.

In a sixteenth aspect of the present disclosure, which may be used in combination with the fourteenth aspect, the first and second portions are configured to be connected such that that (i) the housing coming into operational communication with the selector and (ii) the rotor coming into operational communication with the motor occur along a single axis of movement.

In a seventeenth aspect of the present disclosure, which may be used in combination with any one or more of the preceding aspects, the infusion pump is configured such that the housing (i) in a first position is operable to gravity feed a drug to a patient and (ii) in a second position is operable with the rotor and the motor to pump the drug to the patient.

In an eighteenth aspect of the present disclosure, which may be used in combination with the seventeenth aspect, the second position is obtained rotationally from the first position.

In a nineteenth aspect of the present disclosure, which may be used in combination with the seventeenth aspect, the second position is obtained translationally from the first position.

In a twentieth aspect of the present disclosure, which may be used in combination with the seventeenth aspect, the infusion pump is provided with the housing initially in the first position.

In a twenty-first aspect of the present disclosure, which may be used in combination with any one or more of the preceding aspects, the infusion pump is coupled directly to a solution bag.

In a twenty-second aspect of the present disclosure, which may be used in combination with the twenty-first aspect, the infusion pump is configured such that the housing (i) in a first position is operable to gravity feed a drug to a patient and (ii) in a second position is operable with the rotor and the motor to pump the drug to the patient.

In a twenty-third aspect of the present disclosure, which may be used in combination with any one or more of the preceding aspects, an infusion pump includes a housing; a rotor inserted into the housing, the rotor including a first ring of surfaces that form channels with the housing, the first channel forming surfaces sized to produce a first flowrate, and a second ring of surfaces that form channels with the housing, the second channel forming surfaces sized to produce a second flowrate different than the first flowrate; a motor operably engaged to rotate the rotor; and a selector moveable to allow the rotor to rotate so as to achieve at least one of the first and second flowrates.

In a twenty-fourth aspect of the present disclosure, which may be used with the twenty-third aspect in combination with any of the preceding aspects, the selector is moveable to allow the rotor to pump the first flowrate or a combined flowrate from the first and second flowrates.

It is accordingly an advantage of the present disclosure to provide a relatively low cost, ambulatory infusion pump.

It is another advantage of the present disclosure to provide a variable flowrate ambulatory infusion pump.

It is a further advantage of the present disclosure to provide an ambulatory infusion pump that can dampen pulsatile flow.

It is yet another advantage of the present disclosure to provide an ambulatory infusion pump that is selectable between a gravity flow mode and a pumping mode.

It is still a further embodiment of the present disclosure to provide an ambulatory infusion pump that can be connected directly to an intravenous ("IV") infusion bag.

Additional features and advantages are described herein, and will be apparent from the following Detailed Description and the figures.

### BRIEF DESCRIPTION OF THE FIGURES

Fig. 1 is a partially sectioned plan view of one embodiment of a rotary ambulatory infusion pump of the present disclosure.
Fig. 2 is a plan view of one embodiment of a reusable portion for the rotary ambulatory infusion pump embodiment of Fig. 1.
Fig. 3 is a side-sectioned view of for the rotary ambulatory infusion pump embodiment of Fig. 1.
Fig. 4 is a plan view of a second embodiment of a rotary ambulatory infusion pump of the present disclosure.
Fig. 5 is a side-sectioned view of the rotary ambulatory infusion pump embodiment of Fig. 4.
Fig. 6 is a top plan of the rotary ambulatory infusion pump embodiment of Fig. 4.
Fig. 7 is a top plan view of one embodiment of a rotor of the present disclosure having staggered chamber-forming rotor surfaces for non-pulsatile flow.
Figs. 8 and 9 are sectioned elevation views illustrating one embodiment of a rotary ambulatory infusion pump of the present disclosure providing pumped flow in selectable combination with gravity fed flow.
Figs. 10 and 11 are sectioned elevation views illustrating a second embodiment of a rotary ambulatory infusion pump of the present disclosure providing pumped flow in selectable combination with gravity fed flow.
Figs. 12 and 13 are sectioned elevation views illustrating a third embodiment of a rotary ambulatory infusion pump of the present disclosure providing pumped flow in selectable combination with gravity fed flow.
Figs. 14 and 15 are top plan views illustrating a fourth embodiment of a rotary ambulatory infusion pump of the present disclosure providing pumped flow in selectable combination with gravity fed flow.
Fig. 16 is a sectioned elevation view illustrating one embodiment of a system of the present disclosure in which an intravenous ("IV") bag is directly coupled to a rotary ambulatory infusion pump.
Figs. 17 and 18 are sectioned elevation views illustrating a second embodiment of a system of the present disclosure in which an intravenous ("IV") bag is directly coupled to a rotary ambulatory infusion pump, here providing gravity flow capability.
Figs. 19 and 20 are sectioned elevation views illustrating a third embodiment of a system of the present disclosure in which an intravenous ("IV") bag is directly coupled to a rotary ambulatory infusion pump, here again providing gravity flow capability.

### DETAILED DESCRIPTION

### Slide Operated

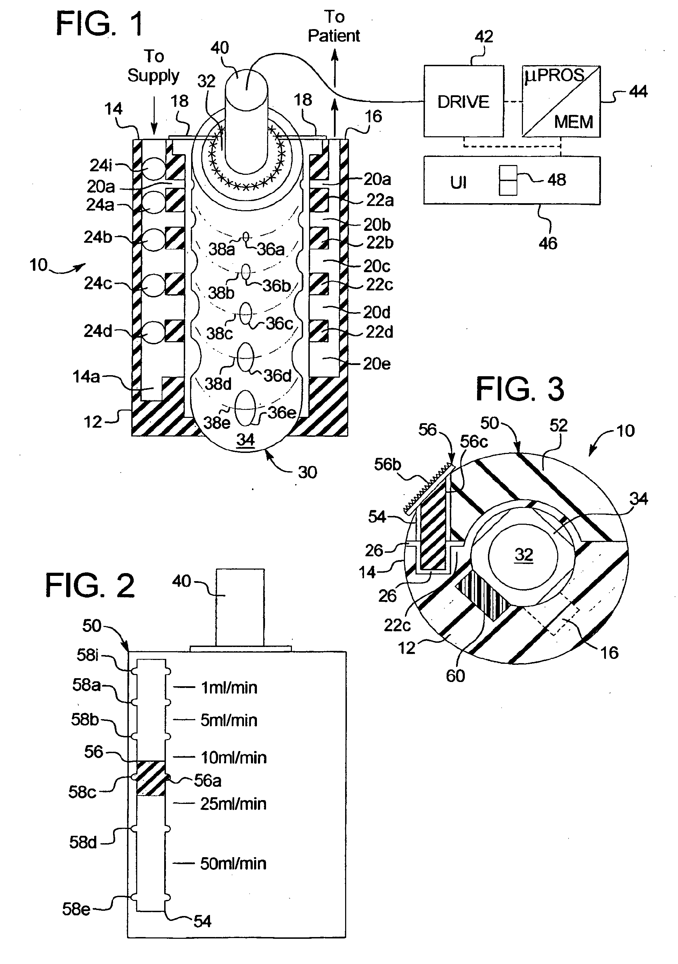

The present invention relates to an infusion pump according to claim 1. Referring now to the drawings and in particular to Figs. 1 to 3, pump 10 illustrates an infusion pump of the present disclosure. U.S. Patent No. 7,674,100 ("the ' 100 Patent"), assigned to PDD Innovations Ltd, teaches for example at its Figs. 1 to 4 that a pump is formed by a housing (10) having an inlet (11) for connection to a source of fluid and an outlet (12) for pumped fluid. A rotor (15) is rotatable within the housing, and the inlet (11) and the outlet (12) are spaced apart around the path of the rotor (15) in the housing. The rotor (15) has surfaces (16*a*, 16*b*, 16c, 16*d*) that form, with the housing (10), closed chambers (18*a*, 18*b,* 18c, *18d*)*,* which travel around the housing (10) to convey fluid from the inlet (11) to the outlet (12). The housing (10) carries a seal (14) that is located between the inlet (11) and the outlet (12) in the direction of travel of the rotor (15). The seal (14) cooperates with the rotor surfaces (16*a*, 16*b*, 16*c*, 16*d*) as the surfaces (16*a*, 16*b*, 16c, 16*d*) pass between the outlet (12) and the inlet (11) to prevent the formation of a chamber during said passage and to prevent fluid flow from the outlet (12) to the inlet (11). Such a pump is easily and cheaply produced.

Applicants' pump 10 as seen in present Fig. 1 likewise includes a housing 12 and a rotor 30 fitted sealingly and removably within housing 12. Housing 12 in an embodiment is a disposable piece that is discarded after a single use or multiple successive uses, e.g., until a supply of a drug is exhausted, and can be made for example from any suitable medically accepted plastic, such as polyvinylchloride ("PVC") or silicone. Disposable housing 12 includes or defines an inlet 14, which can connect to a drug inlet tube (not illustrated), leading to a supply of the drug (not illustrated). Disposable housing 12 includes or defines an outlet 16, which can connect to a drug outlet tube (not illustrated), leading to a patient connection, e.g., a needle, catheter or cannula (not illustrated).

Inlet 14 and outlet 16 are connected at all times to the inlet tube, which is connected at all times to the drug supply (not illustrated) and outlet tube, which is connected at all times to the patient until therapy is completed (not illustrated), such that there is no chance for the drug's sterility to be compromised at any intervening connecting point. In an embodiment, rotor 30 is also a disposable piece and is sealed within housing 12 via a membrane or cover 18. Membrane or cover 18, which can be flexible, semi-rigid and/or rigid, is sealed to a non-rotating inner collar 32 of rotor 30, so as to completely envelop and maintain the sterility of the fluid drug pumped through pump 10.

Disposable housing 12 defines flowpaths 20a to 20e, which communicate fluidly with rotor rings 38a to 38e, respectively, having flattened chamber-forming rotor surfaces 36a to 36e, respectively, similar to the rotor surfaces (16*a*, 16*b*, 16c, 16*d*) of the '100 Patent. While five flowpaths 20a to 20e and rotor rings 38a to 38e are illustrated, pump 10 can have any suitable number of such pairs. Housing 12 also defines a notch 14a that forms part of inlet pathway 14 to allow a valve closing mechanism or occluder 56 to slide into the notch for a desired fully opened flow as discussed in more detail below.

Flowpaths 20a to 20e are separated by divider walls 22a to 22d, respectively, formed with disposable housing 12. Valve areas or valve seats 24a to 24d are located directly adjacent to divider walls 22a to 22d. An initial valve area or valve seat 24i is located just upstream from flowpath 20a. Valve areas 24i and 24a to 24d are areas of a flexible membrane 26 (Fig. 3), one of which at a given time is selected by the operator to be compressed by valve closing mechanism or occluder 56 to block flow to none, one or more of flowpaths 20a to 20e. Flexible membrane 26 (Fig. 3) can be of any medically accepted stretchable polymers, such as flexible PVC or silicone sheeting, and can be ultrasonically welded, solvent bonded or heat sealed to disposable housing 12 as desired.

Rotor 30 includes a head 34 that rotates about stationary collar 32, which is sealed to housing 12 via cover 18. Head 34 defines differently sized flattened surfaces 36a to 36e, which are located along surface rings 38a to 38e. In the illustrated embodiment, each ring 38 (referring collectively to rings 38a to 38e or individually but generally to any ring) includes four flattened surfaces 36 (referring collectively to flattened surfaces 36a to 36e or individually but generally to any flattened surface), however, the rings 38 could alternatively include two, three or more than four flattened surfaces 36.

A motor 40 includes a shaft (not illustrated) that extends through stationary collar 32 and locks removably to rotor head 34 (e.g., via spring-loaded bearings that snap into detent recesses located on the inside of rotor head 34, not illustrated), so that motor 40 can turn head 34. In an embodiment, motor 40 is a low cost, single speed, single direction motor, such as an AC or DC motor. Motor 40 receives current from a motor drive 42, which can be controlled via processing and memory 44, which in turn communicates with a user interface 46. User interface 46 can include a video readout if desired, and one or more electromechanical input device, such as membrane switches or a touch screen.

In one low cost embodiment, user interface 46 includes an on/off switch 48 and informative indicia (see Fig. 2), such that it may be possible to eliminate processing and memory 44. Here, on/off switch 48 allows or does not allow power to be applied to motor drive 42, which in turn powers or does not power motor 40.

Each of the just-described control structures and alternatives is applicable to each of the embodiments of the rotary ambulatory infusion pump described herein. The control structures and alternatives are accordingly not repeated for other embodiments herein employing motor 40 or other motion control.

Fig. 2 illustrates a reusable component 50 of pump 10. Reusable component 50 includes a reusable housing 52, to which motor 40, drive 42, processing and memory 44 (if needed) and user interface 46 (e.g., switch 48) are mounted for repeated use. Although not illustrated, a plug or cord for outside power and/or a battery supply is also carried by housing 52. One suitable battery operated infusion pump system is set forth in U.S. Patent Application No. 12/573,620, filed October 5, 2009, entitled "Infusion Pump With Battery Operation Capability". When motor 40 carried by reusable component 50 locks into rotary head 34 for operation, reusable housing 52 correspondingly slides into operable position with disposable housing 12, namely, so that a valve closing mechanism is positioned properly with valve seat 24i and 24a to 24d of disposable housing 12.

Each of the just-described structures and alternatives for reusable component 50 is applicable to each of the embodiments of the rotary ambulatory infusion pump described herein that uses a motor 40. The structures and alternatives for reusable component 50 are accordingly not repeated for further embodiments employing the reusable component.

Reusable housing 52, which can be made of any of the materials described for housing 12, or of a more rugged material, such as metal, defines an elongated slot 54, within which the operator (e.g., patient, nurse or clinician) moves a valve closing mechanism, selector or occluder 56 (shown sectioned in Fig. 2) mentioned above. Detent hub recesses 58i and 58a to 58d are located along the elongated sides of slots 54 to lock detent hubs 56a of valve closing mechanism or occluder 56 in place at a location correspondingly above valve seats 24i and 24a to 24d, respectively. An additional detent hub recess 58e is located to likewise removably hold valve closing mechanism 56 in place in the fully opened flow position when the valve closing mechanism rests inside notch 14a of inlet pathway. The user slides valve closing mechanism 56 vertically downwardly (in the illustrated orientation of Fig. 2) into the different detent recesses 58a to 58e to increase drug flow. The user slides valve closing mechanism 56 vertically upwardly (in the illustrated orientation of Fig. 2) into the different detent recesses 58i and 58a to 58d to decrease or stop drug flow.

As seen in Fig. 3, valve closing mechanism 56 includes a knurled or otherwise textured tab 56b, which the user grasps to move the valve closing mechanism. A stem portion 56c extends downwardly from tab 56b, through elongated slot 54, and into inlet pathway 14 of disposable housing 12: Wherever stem portion 56c is slid within slot 54 and pathway 14, the stem pushes the corresponding portion of flexible membrane 26 to the bottom of into flowpath 14 to close off flow at that location. In Fig. 1, outlet pathway 16 is shown located one-hundred-eighty degrees from inlet pathway 14 around rotary head 34, which helps to illustrate pump 10. In Fig. 3, outlet pathway 16 is shown alternatively to be angularly closer to inlet pathway 14. Likewise, a seal 60 is located in disposable hosuing 12 between inlet pathway 14 and outlet pathewya 16, which can be a compressible seal of a suitable medical grade material, such as silicone.

Flattened surfaces 36a to 36e are sized and numbered along surface rings 38a to 38e, respectively, to allow for a certain flow through a certain flowpath 20a to 20e, respectively, for a given single, constant speed of motor 40. In this manner, a low cost motor 40 can be used to achieve desired varying drug flowrates. This in combination with the manual control of valve closing mechanism or occluder 56 and on/off switch 48 is believed to provide a low cost but effective ambulatory infusion pump 10. In the illustrated embodiment, flattened surfaces 36a are sized and numbered to provide a drug flowrate of one milliliter ("ml") per minute if desired and if hubs 56a of valve closing mechanism 56 is slid to lock into detent recess 58a. Flattened surfaces 36b are sized and numbered to provide a drug flowrate of four ml/minute, which in combination with the one ml/minute through flowpath 20a provides a total flowrate of five ml/minute if desired and if valve closing mechanism 56 is slid to lock into detent recesses 58b. Flattened surfaces 36c are sized and numbered to provide a drug flowrate of five ml/minute, which in combination with the one ml/minute through flowpath 20a and the four ml/minute through flowpath 20b provides a total flowrate of ten ml/minute if desired and if valve closing mechanism 56 is slid to lock into detent recesses 58c. Flattened surfaces 36d are sized and numbered to provide a drug flowrate of fifteen ml/minute, which in combination with the one ml/minute through flowpath 20a, the four ml/minute through flowpath 20b, and the five ml/minute through flowpath 20c provides a total flowrate of twenty-five ml/minute if desired and if valve closing mechanism or occluder 56 is slid to lock into detent recesses 58d. Finally, flattened surfaces 36e are sized and numbered to provide a drug flowrate of twenty-five ml/minute, which in combination with the one ml/minute through flowpath 20a, the four ml/minute through flowpath 20b, the five ml/minute through flowpath 20c, and the fifteen ml/minute through flowpath 20d provides a total flowrate of fifty ml/minute if desired and if valve closing mechanism 56 is slid to lock into the last detent recesses 58e.

In the illustrated embodiment, flattened surfaces 36a to 36e are accordingly sized and numbered to provide a total drug flowrate of one ml/hour, five ml/hour, ten ml/hour, twenty-five ml/hour, or fifty ml/hour or any other suitable or desired flowrates. It should be appreciated however that flattened surfaces 36a to 36e can be sized and numbered to allow for other suitable and desired flowrates.

Pump 10 can idle if on/off switch 48 is left on and valve closing mechanism 56 is slid to lock into the initial detent recess 58i. To shut pump 10 off, the user shuts on/off switch 48 to the off position in one embodiment. Rotor head 34 acts as a closed valve or occluder when stationary. Closing mechanism or occluder 56 and valve seats 24i and 24a to 24d can be located alternatively or additionally in outlet pathway 16 if desired.

While infusion pump 10 uses flatted portions 36 of different sizes to set the desired flowrates, e.g., one, five, ten, twenty-five, and fifty ml/min, it should be appreciated that other rotary, ambulatory structures could be used instead. For example, a multi-finger operated rotary ambulatory infusion pump is contemplated to provide user controlled flowrates, wherein a user actuates one finger to choose one flow rate or actuates multiple fingers to choose a combination of flow rates. In one example, the fingers are actuated by the user pressing a button.

### Dial Operated

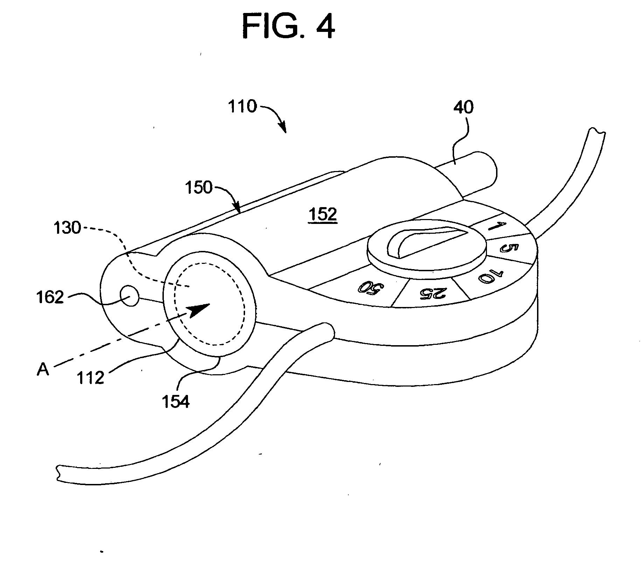

Referring now to Figs. 4 to 6, an alternative dial operated rotary ambulatory infusion pump 110 is illustrated. Fig. 4 illustrates one embodiment of a reusable component 150, which includes a clamshell type housing 152 that encloses a disposable housing 112. Clamshell housing 152 defines an aperture 154 that allows a rotor 130 to lock onto the shaft of motor 40 as discussed above, and for disposable housing 112 to be loaded into clamshell housing 152 in a single step from a direction indicted by arrow A. Reusable component 150 and disposable housing 152 can be made of any of the materials described above for pump 10.

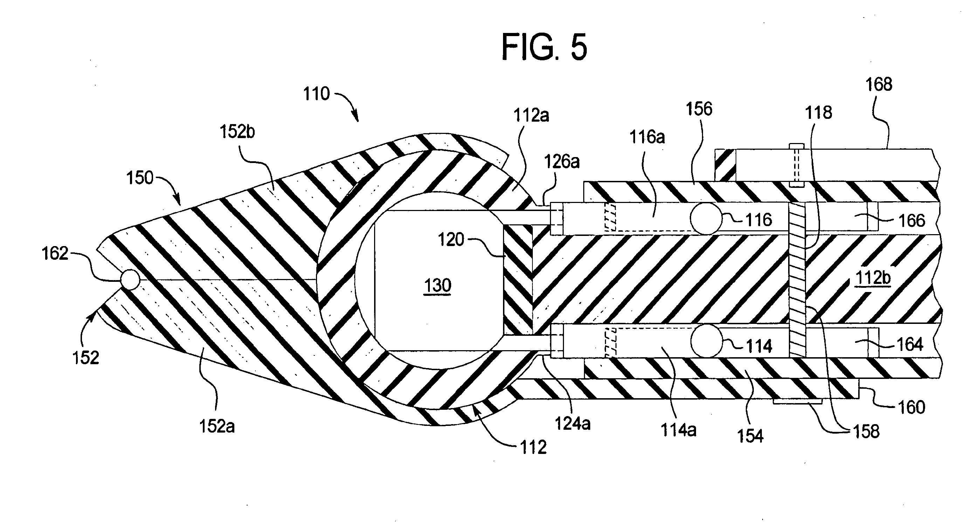

Fig. 5 illustrates that disposable housing 112 includes an annular portion 112a, enclosing rotor 130, and a tube guiding portion 112b. Annular portion 112a houses a seal 120 between inlet pathways 124 and outlet pathewys 126, which is analogous to seal 60 of pump 10. It should be appreciated that seal 120 (as with seal 60) can be a contiguous flexible seal used for each of chamber-forming rotor surfaces 136a to 136e. Alternatively, seal 120 is split into separate flexible seals (e.g., 120a to 120e, not illustrated), which are each sized for operation with the corresponding size of chamber-forming rotor surfaces 136a to 136e of rings 138a to 138c, respectively. For example, seal 120b would be larger than seal 120a, seal 120c would be larger than seal 120b, seal 120d would be larger than seal 120c, and seal 120e would be larger than seal 120d.

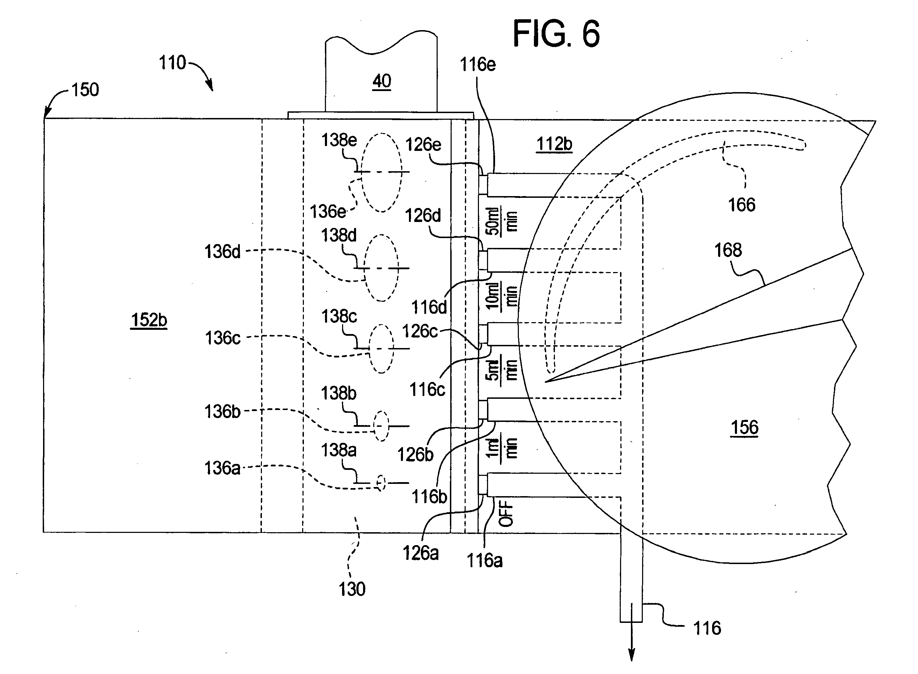

Tube guiding portion 112b (i) guides inlet tubes 114a to 114e (only 114a shown in side view of Fig. 5) to sealingly connect with corresponding inlet ports 124a to 124e (only 124a shown side view of Fig. 5) extending from annular portion 112a of disposable housing 112 and (ii) guides outlet tubes 116a to 116e (all shown in top view of Fig. 6) to sealingly connect with corresponding outlet ports 126a to 126e (all shown in top view of Fig. 6) extending from annular portion 112a of disposable housing 112. Inlet tubes 114a to 114e connect to an overall inlet manifold 114, which connects fluidly and sealingly to a supply of a drug (not illustrated). Outlet tubes 116a to 116e connect to an overall outlet manifold 116, which connects fluidly and sealingly to a patient catheter, needle or cannula (not illustrated).

In Fig. 5, the outlet tubing is coordinated on the top of tube guiding portion 112b, while the inlet tubing is coordinated on the bottom of tube guiding portion 112b. In an alternative embodiment, the tubing of outlet tubing is coordinated on the top of tube guiding portion 112b is replaced by flowpaths molded into tube guiding portion 112b, and path occluding is done alternatively by moving a stretchable membrane similar to the operation of pump 10.

Tube guiding portion 112b of disposable housing 112 also defines an aperture 118 that, for example snap-fittingly, receives a rotating pin 158 fixed to lower rotating occluder dial 154 and upper rotating occluder dial or selector 156, all of reusable component 150. Rotating pin 158 is held rotatably fixed to extension plate 160, which extends from clamshell housing 152, so as to hold lower rotating occluder dial 154 and upper rotating occluder dial 156 in a single arrangement with clamshell housing 152. It should be appreciated that lower rotating occluder dial 154 and upper rotating occluder dial 156 rotate with respect to disposable housing 112, clamshell housing 152, and extension plate 160.

Clamshell housing 152 is provided with a hinge 162 that allows lower clamshell 152a to be hinged away from upper clamshell 152b when (i) disposable housing 112 is fitted to motor 40, which is fixed to lower clamshell 152a, and (ii) tube guiding portion 112b is fitted or snapped onto rotating pin 158. Upper clamshell 152b is then closed over disposable housing 112 to help stabilize rotor 130 during operation.

Lower rotating occluder dial 154 and upper rotating occluder dial 156 are provided with, and in an embodiment formed with, semicircular occluders 164 and 166, respectively. Semicircular occluders 164 and 166 in an embodiment extend for a vertical distance less than the outside diameters of tubing 114 and 116 (including all port tubes 1 14a to 114e and 116a to 116e), so as to allow room for compressed tube thicknesses when tubing 114 and 116 is occluded fully. The circumferential length of semicircular occluders 164 and 166 is set in one embodiment to be able to occlude all port tubes 114a to 114e and 116a to 116e when handle 168 is turned counterclockwise to the "off" position, which is printed or formed into stationary tube guiding portion 112b in the illustrated embodiment. It should be appreciated however that in the "off" position, if manifold tubing 114 and 116 is occluded then all flow is stopped even if some of port tubes 114a to 114e and 116a to 116e remain open, such that semicircular occluders 164 and 166 may be shortened and/or customized, e.g., provided with individual teeth for individual tubes at certain positions of handle 168.

As illustrated in Fig. 6, when handle 168 points to the "off" position, all tubing 114 and 116 (including all port tubes 114a to 114e and 116a to 116e) is occluded, so that no flow can flow into or out of pump 10. It may be found that only one of lower occluder 164 or upper occluder 166 is needed, in which case it is contemplated to provide only the desired inlet or outlet occluder 164 or 166. Although not illustrated, detent recesses (formed, e.g., on top and bottom of tube guiding portion 112b) can receive a mating detent hub (formed, e.g., one each of the upper surface of lower rotating occluder dial 154 and one on the lower surface of upper rotating occluder dial 156) so as to releasably hold rotating occluder dials 154 at a desired one of the set flow positions.

When the user rotates handle 168 from the "off" position to the "1 ml/min" position, inlet tube 114a and outlet tube 116a, communicating fluidly with the one ml/minute producing chamber-forming rotor surfaces 136a, are allowed to open, such that the constant angular speed of rotor 130 produces the desired flow of one ml/minute. When the user rotates handle 168 from the "1 ml/min" position to the "5 ml/min" position, inlet tube 114b and outlet tube 116b, communicating fluidly with four ml/minute producing chamber-forming rotor surfaces 136b, are also allowed to open, such that the constant angular speed of rotor 130 produces the desired overall flow of five ml/minute (all previously opened tubes remaining open). When the user rotates handle 168 from the "5 ml/min" position to the "10 ml/min" position, inlet tube 114c and outlet tube 116c, communicating fluidly with five ml/minute producing chamber-forming rotor surfaces 136c, are allowed to open, such that the constant angular speed of rotor 130 produces the desired overall flow of ten ml/minute (all previously opened tubes remaining open). When the user rotates handle 168 from the "10 ml/min" position to the "25 ml/min" position, inlet tube 114d and outlet tube 116d, communicating fluidly with fifteen ml/minute producing chamber-forming rotor surfaces 136d, are allowed to open, such that the constant angular speed of rotor 130 produces the desired overall flow of twenty-five ml/minute (all previously opened tubes remaining open). Finally, when the user rotates handle 168 from the "25 ml/min" position to the "50 ml/min" position, inlet tube 114e and outlet tube 116e, communicating fluidly with twenty-five ml/minute producing chamber-forming rotor surfaces 136e, are allowed to open, such that the constant angular speed of rotor 130 produces the desired overall flow of fifty ml/minute (all previously opened tubes remaining open).

### Non-Pulsatile Flow

With either pump 10 or pump 110, it is contemplated to stagger chamber-forming rotor surfaces 36 and 136 (referring collectively to surfaces 36a to 36e and 136a to 136e, respectively) in the different rings to produce a more continuous flow as more and more of the flowpaths are opened for higher flowrates. For example, in Fig. 1, the four of each of surfaces 36a to 36e are all facing the same four directions, that is the axis lines orthogonal to surfaces 36a to 36e, separated by ninety degrees, are all aligned. It is contemplated for example to rotate surfaces 36b, such that they are forty-five degrees out of phase with surfaces 36a. Thus when the user increases flow from one ml/minute to five ml/minute, the flow becomes more continuous, because there are now eight output strokes per full revolution of rotor 30 as opposed to four. Similarly, surfaces 36c can be rotated 22.5 degrees, so as to be 22.5 degrees out of phase with surfaces 36a and 36c. Here, when the user increases flow from five ml/minute to ten ml/minute, the flow becomes even more continuous, because there are now twelve output strokes per full revolution of rotor 30 as opposed to eight. Further, surfaces 36d can be rotated 67.5 degrees, so as to be 22.5 degrees out of phase with surfaces 36a and 36c, and forty-five degrees out of phase with surfaces 36b. Here, when the user increases flow from ten ml/minute to fifteen ml/minute, the flow becomes still even more continuous, because there are now sixteen output strokes per full revolution of rotor 30 as opposed to eight. Because surfaces 36d and 36e provide the largest amount of the overall flowrate upon full flow, it is contemplated to rotate surfaces 36e so as to be ninety degrees out of phase with surfaces 36d, which would cause surfaces 36e to be aligned with surfaces 36c in the above example. Nevertheless, flow pulsatility would be further dampened.

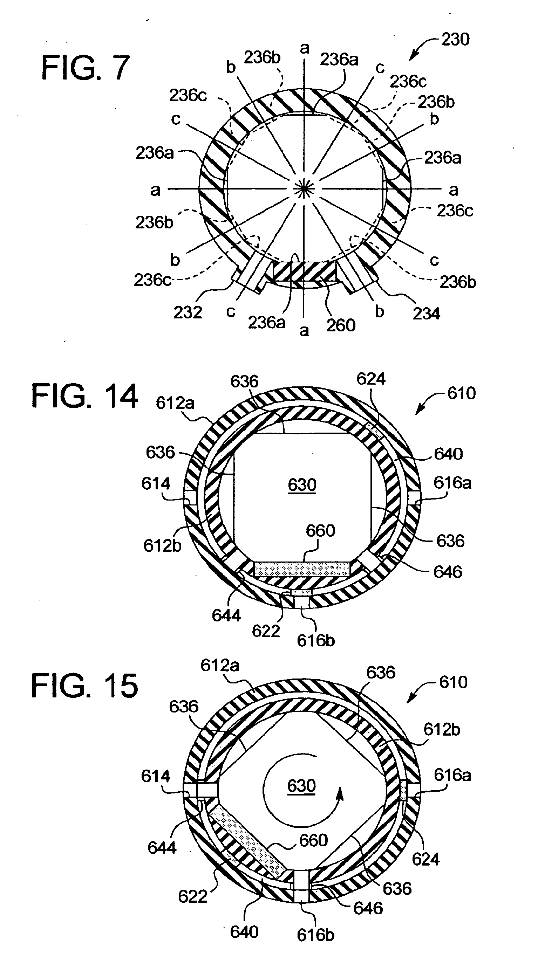

Staggering chamber-forming rotor surfaces is not limited to when the different rings of surfaces have different, e.g., increasing, sizes as has been shown with pumps 10 and 110. Fig. 7 illustrates alternative rotor 230, in which each ring or layer of chamber-forming rotor surface includes surfaces of the same size. Rotor 230, which can be made of any of the materials described herein, includes an inlet 232 and an outlet 234 for each ring or level of chamber-forming rotor surfaces. Rotor 230 also houses a seal 260, e.g., compressible plastic or silicone, which can be contiguous for each ring or level of chamber-forming rotor surfaces or be provided as a separate seal for each chamber-forming rotor surface. The illustrated inlet 232 and outlet 234 operate with the uppermost level of chamber-forming rotor surfaces 236a, which are illustrated as currently being orthogonal to axis a-a. A non-illustrated inlet (such as 232) and outlet (such as 234) operate with an intermediate (into the page) ring or level of chamber-forming rotor surfaces 236b, which are illustrated as currently being orthogonal to axis b-b. A further non-illustrated inlet (such as 232) and outlet (such as 234) operate with a lower-most (into the page) level of chamber-forming rotor surfaces 236c, which are illustrated as currently being orthogonal to axis c-c.

In Fig. 7, chamber-forming rotor surfaces 236b are out of phase with chamber-forming rotor surfaces 236a by thirty degrees. Chamber-forming rotor surfaces 236c are also out of phase with chamber-forming rotor surfaces 236b by thirty degrees. Chamber-forming rotor surfaces 236a are likewise out of phase with chamber-forming rotor surfaces 236c by thirty degrees. Thus even though only three rings of chamber-forming rotor surfaces 236a to 236c are provided, and the size of surfaces 236a to 236c is relatively small, outlets 234 for each axes a-a, b-b, and c-c provide in combination a virtual continuous flow. It is contemplated to use the rotor of Fig. 7 in either the case where (i) the drug is always flowing through the channels of each axis a-a, b-b, and c-c; or (ii) the channels of axes a-a, b-b, and c-c are valved or capable of being occluded as with pumps 10 and 110.

### Pumped Flow In Selectable Combination With Gravity Fed Flow

In many drug infusion applications, it is desirable to let gravity feed the drug to the patient. Gravity flow is in many cases sufficient for the drug and its purpose. Gravity flow is advantageous because the lack of a pump results in a system that will not over-pressurize the patient, that is, there is no pump to attempt to fight through a blockage or occlusion. Each of the pumping systems shown below providing pumped flow in selectable combination with gravity fed flow is shown with a single pump flowpath for ease of illustrating the pump and gravity combination. It should be appreciated however that any of the pumping systems shown below providing pumped flow in selectable combination with gravity fed flow can be combined alternatively with the multi-flowpath, variable flow embodiments discussed above, including each of their alternatives.

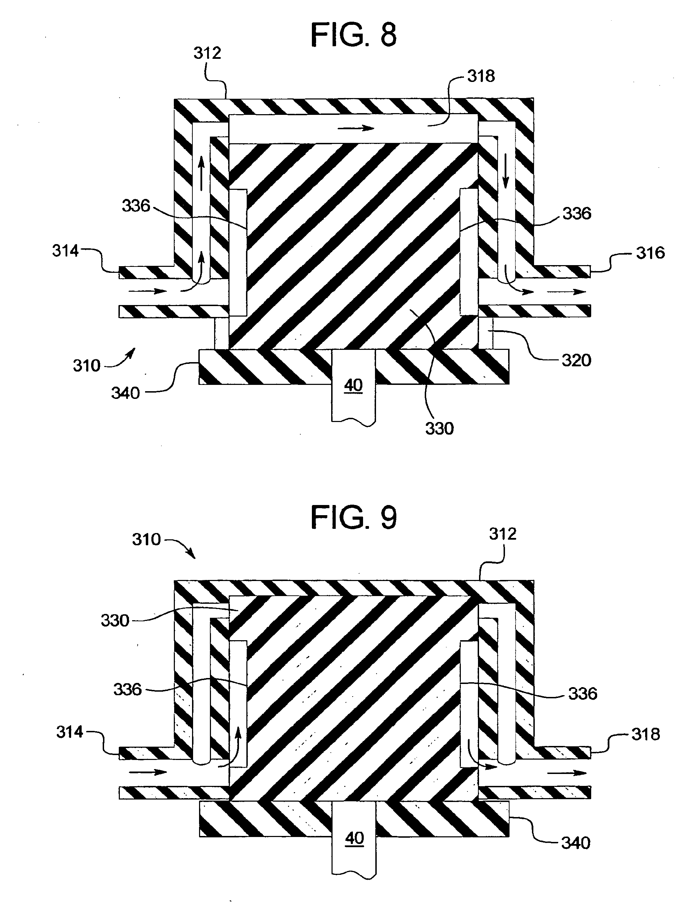

Referring now to Figs. 8 and 9, rotary ambulatory infusion pump 310 illustrates one embodiment of a pumping system providing pumped flow in selectable combination with gravity fed flow. Infusion pump 310, which can be made of any of the materials described herein, includes a disposable housing 312 having an inlet 314 for connecting to a source of a drug and an outlet 316 for connecting to the patient. Disposable housing 312 is fitted tightly to disposable rotor 330 having chamber-forming rotor surfaces 336 for rotary pumping of a drug as has been described in detail above.

Infusion pump 310 also provides a retention wall 340, which can be part of disposable housing 312 or part of the reusable portion of infusion pump 310, e.g., the wall to which motor 40 (not illustrated) is mounted. In either case, a tear-off type o-ring spacer 320 is provided to initially hold housing 312 (at least the part of housing 112 illustrated to include inlet 314 and outlet 316) in a spaced apart manner with respect to retention wall 340. As seen in Fig. 8, when spacer 320 is in place and housing 312 is spaced apart from retention wall 340, the drug is allowed to gravity flow from the inlet pathway 314, through a gravity flowpath 318, and through outlet pathway 316 to the patient.

As seen in Fig. 9, when the user removes spacer 320, housing 312 can be collapsed onto rotor 330 and abutted against retention wall 340. Although not illustrated, mating detent recesses and hubs can be provided between housing 312 and retention wall 340 to hold housing 312 in the collapsed position. Or, housing 312 can be threaded to retention wall 340 to hold housing 312 in the collapsed position, such that inlet 314 and outlet 316 are in a desired alignment with respect to the rest of infusion pump 310. In any case, as seen in Fig. 9, when housing 312 is collapsed onto rotor 330, (i) gravity-fed pathway 318 is closed, and (ii) chamber-forming rotor surfaces 336 are in fluid communication with inlet 314 and outlet 316 to allow for pumped flow. Here, the drug is pumped to the patient and not gravity fed via rotation of rotor 330.

In an alternative embodiment, housing 312 and rotor 330 are structured such that chamber-forming rotor surfaces 336 are not in fluid communication with the inlet 314 and outlet 316 when spacer 320 is in place spacing apart housing 312 from retention wall 314. Here, the drug cannot be pumped even if pump 310 is mistakenly energized when spacer 320 is in place.

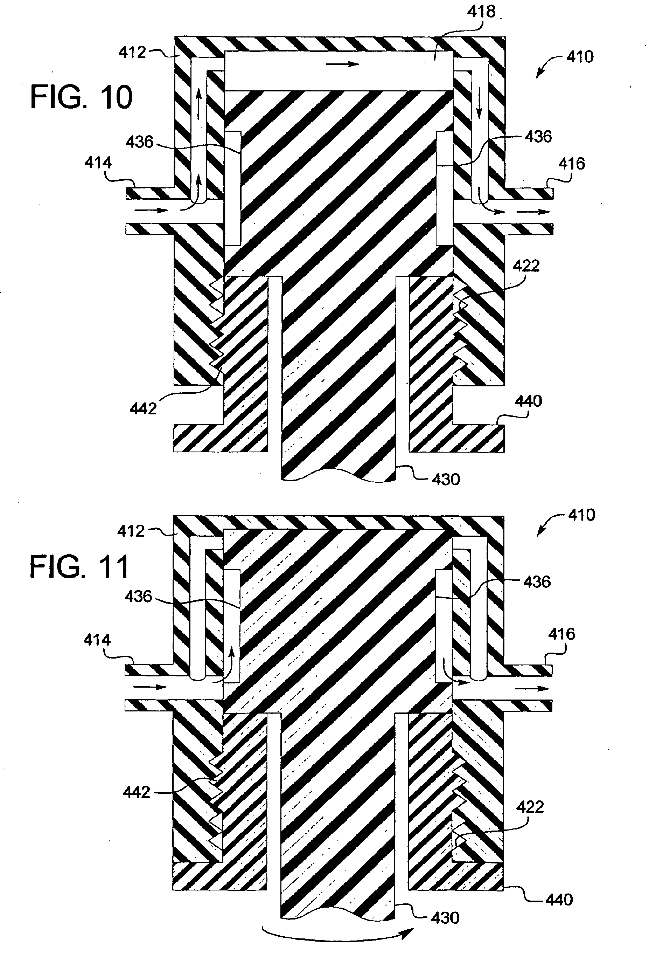

Referring now to Figs. 10 and 11, rotary ambulatory infusion pump 410 illustrates a second embodiment of a pumping system providing pumped flow in selectable combination with gravity fed flow, which operates very similar to pump 310. Infusion pump 410, which can be made of any of the materials described herein, includes a disposable housing 412 having an inlet 414 for connecting to a source of a drug and an outlet 416 for connecting to the patient. Disposable housing 412 is fitted tightly to a disposable rotor 430 having chamber-forming rotor surfaces 436 for rotary pumping of a drug as has been described in detail above.

Infusion pump 410 does not use the retention wall or o-ring spacer of pump 310. Instead, housing 412 is provided with internal threads 422 that mate with external threads 442 of a bushing 440 threaded to housing 412. Bushing 440 in an embodiment is part of the reusable portion of pump 410 and in any case is fixed to the main body of pump 410, such that movement of housing 412 relative to bushing 440 results in a movement of housing 412 relative to the entire pump 410.

As seen in Fig. 10, when housing 412 is in an upper, first translational location relative to bushing 440, the drug is allowed to gravity flow from the inlet pathway 414, through a gravity-fed flowpath 418, through outlet pathway 416 to the patient. As seen in Fig. 11, when housing 412 is rotated to a lower, second translational location relative to bushing 440, (i) gravity pathway 418 is closed, and (ii) chamber-forming rotor surfaces 436 maintain fluid communication with inlet 414 and outlet 416 to allow for pumped flow. Here, the drug is pumped to the patient and not gravity fed.

In an alternative embodiment, housing 412 and rotor 430 are structured such that chamber-forming rotor surfaces 436 are not in fluid communication with the inlet 414 and outlet 416 housing 412 is in the upper, first translational location relative to bushing 440. Here, the drug cannot be pumped even if pump 410 is mistakenly energized when housing 412 is in the upper, first translational location.

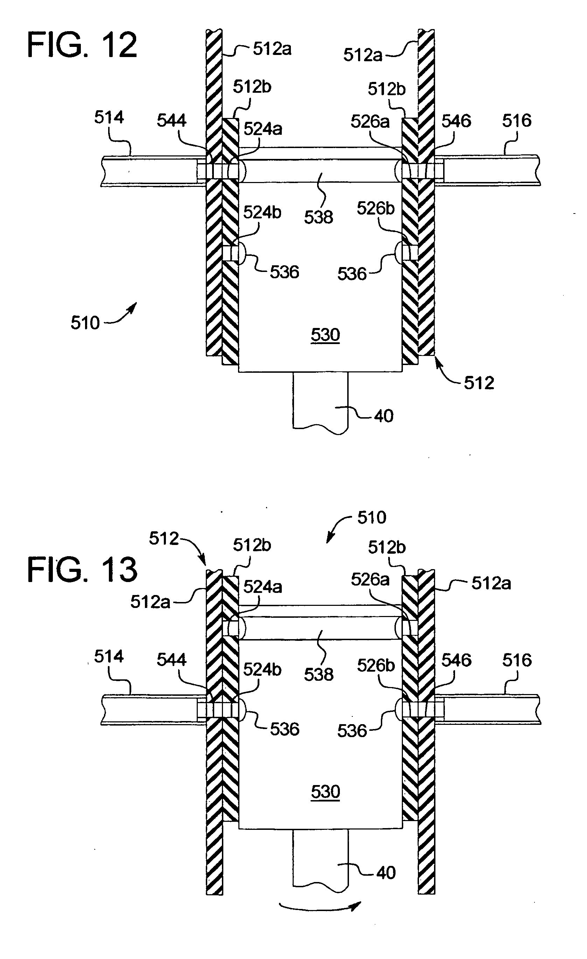

Referring now to Figs. 12 and 13, rotary ambulatory infusion pump 510 illustrates a third embodiment of a pumping system providing pumped flow in selectable combination with gravity fed flow. Infusion pump 510, which can be made of any of the materials described herein, includes a disposable housing 512 having an outer wall 512a connected to an inlet pathway 514 for connecting to a source of a drug and an outlet pathway 516 for connecting to the patient. Disposable housing 512 includes an inner wall 512b, which is compressively sealed to outer wall 512a or is alternatively threaded to outer wall 512a in luer lock type of arrangement.

Disposable inner wall 512b is fitted tightly to disposable rotor 530 having lower chamber-forming rotor surfaces 536 for rotary pumping of a drug as has been described in detail above. Disposable rotor 530 also defines an upper annular ring 538, which extends three-hundred-sixty degrees about rotor 530, such that an inlet 524a of inner wall 512b is always in communication with an outlet 526a of inner wall 512b regardless of the rotational position of rotor 530 with respect to inner wall 512b. Inner wall 512b also defines an inlet 524b and an outlet 526b, which communicate with chamber-forming rotor surfaces 536 when the surfaces are rotated into communication with inlet 524b and outlet 526b.

Disposable outer wall 512a defines in an inlet 544, which communicates with inlet pathway 514, and an outlet 546, which communicates with outlet pathway 516. As seen in Fig. 12, when inner wall 512b is in a first translational state relative to outer wall 512a, the drug is gravity fed from inlet tube 514, through outer wall inlet 544, through inner wall inlet 524a, around annular groove 538, out inner wall inlet 526a, out outer wall inlet 546, and though outlet tube 516 to the patient.

As seen in Fig. 13, when inner wall 512b is translated upwardly relative to outer wall 512a, e.g., via a sliding compression seal movement or via a threaded luer lock seal movement, inner wall inlet 524a, annular groove 538, and inner wall outlet 526a are moved out of fluid communication with, outer wall inlet 544, inlet pathway 514, outer wall outlet 546, and outlet pathway 516. At the same time, inner wall pumping inlet 524b, chamber-forming rotor surfaces 536, and inner wall pumping outlet 526b are moved into fluid communication with outer wall inlet 544, inlet pathway 514, outer wall outlet 546, and outlet pathway 516, such that the drug can now be pumped to the patient. It is contemplated that the compression seal or luer lock seal between outer wall 512a and inner wall 512b is maintained such that pump 510 can be switched back and forth between gravity feeding the drug in Fig. 12 and pumping the drug in Fig. 13 multiple times.

Referring now to Figs. 14 and 15, rotary ambulatory infusion pump 610 illustrates a forth embodiment of a pumping system providing pumped flow in selectable combination with gravity fed flow. Infusion pump 610, which can be made of any of the materials described herein, includes a disposable housing having an outer wall 612a defining an inlet 614 for connecting to a source of a drug, a first outlet 616a for connecting gravity fed drug flow to the patient and a second outlet 616b for connecting pumped drug flow to the patient.

The disposable housing also includes an inner wall 612b, which is held rotatably fixed within outer wall 612a via stoppers 622 and 624. Inner wall 612b includes a compressible seal 660, as has been described herein, and operates with rotor 630, defining chamber-forming rotor surfaces 636, for pumped flow, as has been described herein. Inner wall 612b also includes or defines pump inlet 644 and pump outlet 646.

In Fig. 14, the drug is gravity fed. Here, outer wall inlet 614 delivers a drug into a space 640 located between inner wall 612b and outer wall 612a and set via stoppers 622 and 624. The fluid flows through space 640, around inner wall 612b and around stopper 624 to gravity flow outlet 616a. In Fig. 14, stopper 622 plugs pumped flow outlet 616b, so no drug flows through that outlet. Also, pump inlet 644 and pump outlet 646 of inner wall 612b are scaled against the inside surface of outer wall 612a.

In Fig. 15, the drug is pumped to the patient. Here, inner wall 612b has been turned approximately forty-five degrees relative to outer wall 612a. Now, pump inlet 644 of inner wall 612b communicates with outer wall inlet 614. Also, pump outlet 646 of inner wall 612b now communicates with outer wall pump outlet 616b. Further, stopper 624 is rotated to plug outer wall gravity flow outlet 616a. Stopper 622 is rotated out of the way. In Fig. 15, when motor 40 drives rotor 630, the drug is pumped through outer wall inlet 614, through inner wall pump inlet 644, is spun by rotor surfaces 636 to inner wall pump outlet 646 and out outer wall pump outlet 616b, and is thereby pumped to the patient.

It should be appreciated that in an alternative embodiment, instead of the rotary ambulatory infusion pump having two outlets, such as first outlet 616a and second outlet 616b shown in Figs. 14 and 15, the rotary ambulatory infusion pump has only one outlet. For example, in one embodiment second outlet 616b, and stoppers 622 and 624 of the rotary ambulatory infusion pump shown in Fig. 15 are removed. In this embodiment, fluid flowing through fluid space 640 is not blocked at pump outlet 646, and can be pumped through pump outlet 646 to the remaining outer wall outlet 616a.

### Direct Coupling To Intravenous ("IV") Bag

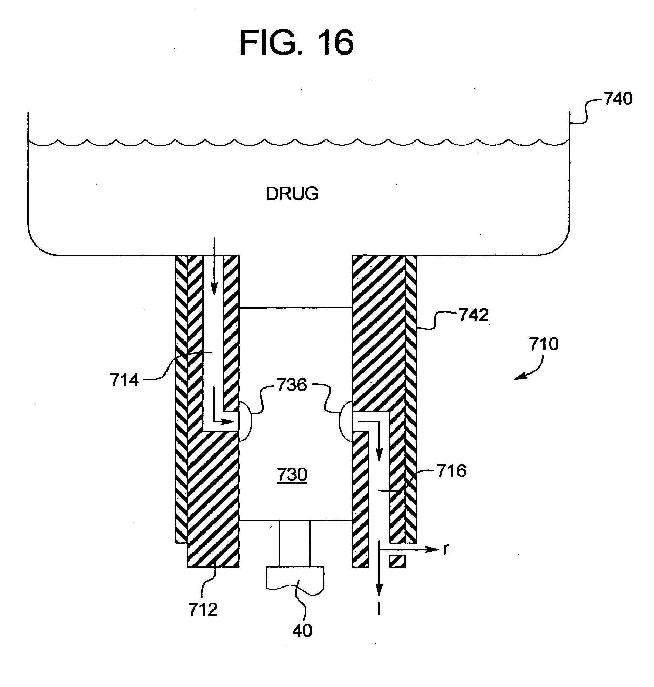

Referring now to Fig. 16, rotary ambulatory infusion pump assembly 710 illustrates one embodiment of a direct coupling of the rotary ambulatory infusion pump to an intravenous ("IV") bag, namely IV bag 740, is illustrated. Bag 740 defines a bag port 742. Housing 712 of pump assembly 710 is permanently (e.g., formed with, welded to, solvent bonded to, or heat sealed to) or releasably (e.g., via a snap-fit) secured to bag port 742. For example, the disposable portion of the pump of pump assembly 710 can be formed with bag port 742, while the reusable portion of pump assembly 710 can be releasably secured to the disposable portion of the pump, for example, via the motor 40 to rotor connection.

Rotor 730 of pump assembly 710, including chamber-forming rotor surfaces 736, is fitted sealingly within housing 712 for pump operation according to teachings set forth repeatedly herein. Inlet pathway 714 leads from bag 740 to chamber-forming rotor surfaces 736. Outlet pathway 716 leads from chamber-forming rotor surfaces 736 to the patient. Fig. 16 illustrates that the patient connection to outlet pathway 716 can extend radially ("r") away from outlet pathway 716 or longitudinally ("I") away from the outlet pathway.

Pump assembly 710 eliminates the inlet tubing from the IV bag to the pump, further reducing cost. Pump assembly 710 also provides an efficient, out of the way location to mount the infusion pump. It should be appreciated that while pump assembly 710 shows the rotary pump having only a single ring of chamber-forming rotor surfaces 736, multiple rows of chamber-forming rotor surfaces 736 could be provided alternatively according to the teachings above for variable flow and/or pulsatility dampening purposes.

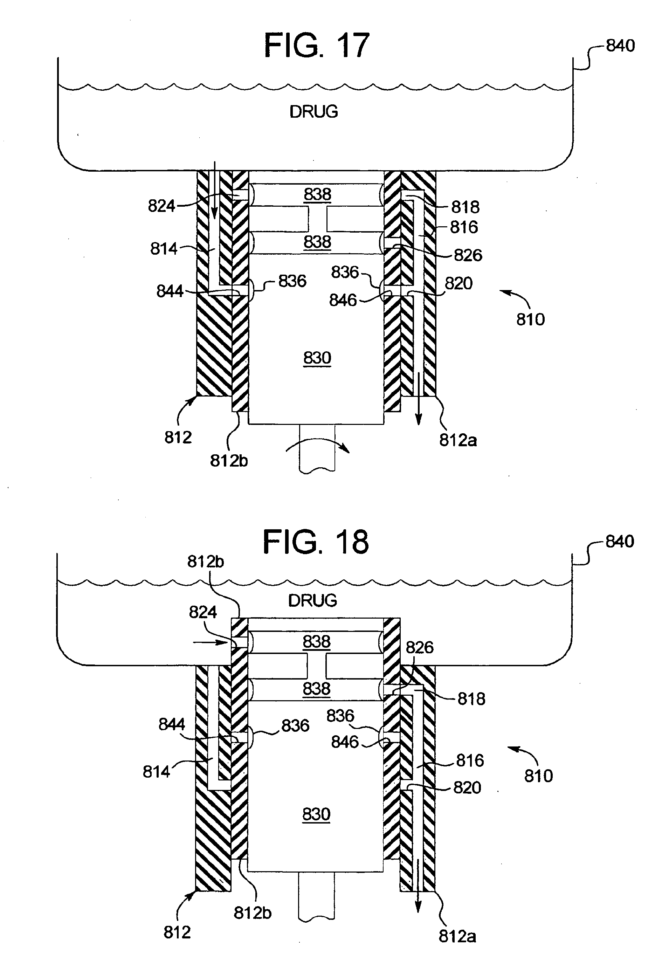

Referring now to Figs. 17 and 18, rotary pump assembly 810 illustrates another embodiment of a direct coupling of a rotary ambulatory infusion pump to an IV bag, and here adds alternative gravity flow capability. Assembly 810 includes a disposable housing 812, which in the illustrated embodiment is formed with or welded to IV bag 840. Housing 812 includes an outer housing portion 812a and an inner housing portion 812b, which is translationally engaged to outer housing portion 812a, e.g., via a sliding compression seal arrangement or a threaded luer lock arrangement discussed above also with respect to pump 510. Outer housing portion 812a defines an inlet pathway 814 and an outlet pathway 816. Inlet pathway 814 communicates fluidly with the drug stored in IV bag 840, which again eliminates the need for separate inlet tubing. Outlet pathway 816 communicates fluidly with the patient, e.g., via a needle, catheter or cannula. Outlet pathway 816 also includes dual passageways 818 and 820 leading to sealed inner housing portion 812b

Inner housing portion 812b defines a first, upper inlet 824 a first, upper outlet 826, a second, lower inlet 844 and a second, lower outlet 846. Rotor 830 fits sealingly inside inner housing portion 812b according to the teachings provided herein repeatedly and moves with inner housing portion 812b. Rotor 830 includes chamber-forming rotor surfaces 836 that are placed in communication with lower inlet 844 and lower outlet 846 when rotor 830 is rotated. Rotor 830 also defines dual annular channels 838 that communicate fluidly via one or more vertical channel. Annular channels 838 spans three-hundred-sixty degrees around rotor 830 and allow upper inlet 824 to communicate with upper outlet 826 regardless of the rotational position of rotor 830.

When the patient desires, or the prescription calls for, pumped drug flow, the patient leaves inner housing 812b in the position of as seen in Fig. 17, wherein inner housing portion 812b is in a lower translational position relative to outer housing portion 812a, such that the drug is pumped from bag 840, through inlet pathway 814 and inlet 844, around rotor 830 via rotation of chamber-forming rotor surfaces 836, out outlet 846 and channel 820 of outlet pathway 816 to the patient. In this lower translational position, drug from bag 840 cannot reach upper inlet 824 of inner housing portion 812b, so that only pumped flow occurs.

When the patient desires, or the prescription calls for, gravity pump flow, the patient leaves or places inner housing 812b in the position shown in Fig. 18, wherein inner housing portion 812b is in an upper translational position relative to outer housing portion 812a, such that inlet 824 and upper annular groove 838 lie inside bag 840, and so that the drug can be gravity fed from bag 840 (i) through inlet 824 of inner housing portion 812b, through annular grooves 838, out outlet 826 and channel 818 of outlet pathway 816 to the patient. In this upper translational position, inlet 844 and outlet 846 of inner housing portion 812b are blocked, so that only gravity flow occurs.

In an embodiment, inner housing portion 812b and rotor 830 break a seal of bag 840 when pushed up into bag 840 as illustrated in Fig. 18. In such a case, inlet 824 and the upper annular groove 838 and vertical groove connecting the upper and lower annular grooves 838 can be removed. Instead, one or more vertical groove (not illustrated) can be provided extending from the lower (remaining) annular groove 838 up through the top of rotor 830. In Fig. 17, the top of the one or more vertical groove is covered by the bag film. When the bag film is pierced in Fig. 18, the drug can gravity flow down the one or more vertical groove into the remaining annular groove 838 and out of housing 812 as described above.

Although not illustrated, inner housing portion 812b and outer housing portion 812a can having mating detent recesses and hubs or end of travel luer lock positions, so as to releasably secure the inner housing portion 812b in the respective positions relative to outer housing portion 812a shown in Figs. 17 and 18.

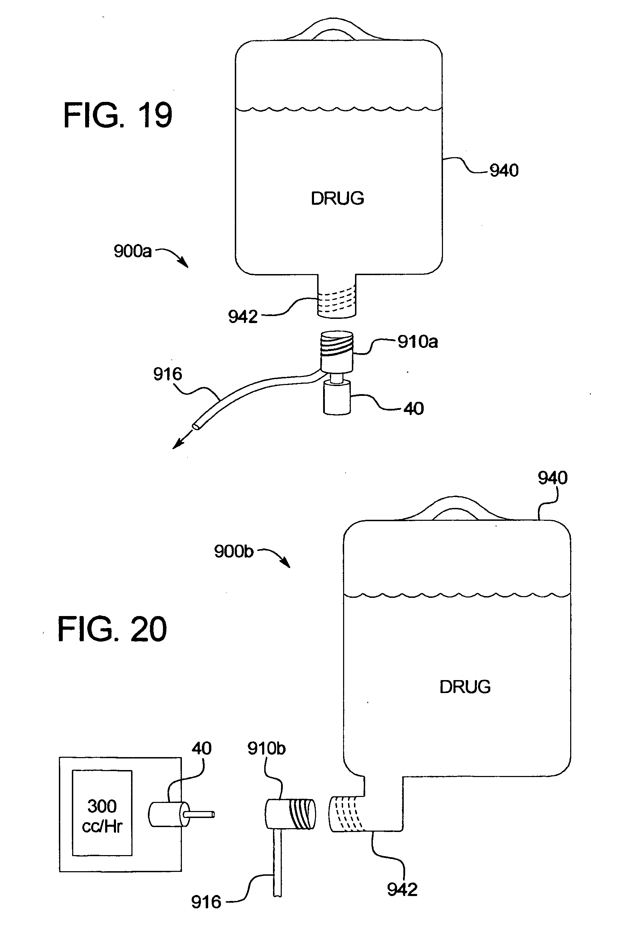

Referring now to Figs. 19 and 20, assemblies 900a and 900b illustrate different combinations for joining the rotary pumps of the present disclosure to an IV bag 940. In assembly 900a of Fig. 19, motor 40 is pre-fitted to pump 910a, which are then both connected to IV bag 940, e.g., via a luer lock connection. In assembly 900b of Fig. 20, the disposable portion of pump 910b is connected first to IV bag 940, e.g., via a luer lock connection. Then, motor 40 is slid into the rotor of pump 910b for operation. As illustrated, both assemblies 900a and 900b pump out to the patient via patient line 916 (similar patient line is provided with each embodiment described herein). Other reusable parts of pump 910b, besides motor 40, can be pre-fitted to the disposable portion of pump 910b or to motor 40 as desired.

## Claims

1. An infusion pump (10, 110, 310, 410, 510, 610, 710, 810, 910a, 910b) comprising:
a housing (12, 112, 312, 412, 512, 612a/612b, 712, 812) defining (i) a plurality of flowpaths (20a, 20b, 20c, 20d, 20e) separated by respective divider walls (22a, 22b, 22c, 22d), (ii) an inlet (14), and (iii) an outlet (16), each of the plurality of flowpaths (20a, 20b, 20c, 20d, 20e) in fluid communication with the inlet (14) and the outlet (16);
a rotor (30, 130, 230, 330, 430, 530, 630, 730, 830) inserted into the housing, the rotor including a first ring (38a, 138a) of surfaces (36a, 136a, 236a, 336, 436, 536, 636, 736, 836) that form channels with a first one of the plurality of flowpaths of the housing, and a second ring (38b, 138b) of surfaces (36b, 136b, 236b, 336, 436, 536, 636, 736, 836) that form channels with a second one of the plurality of flowpaths of the housing, the second channel forming surfaces radially offset from the first channel forming surfaces;
a plurality of valve seats (24a, 24b, 24c, 24d, 24i) comprising areas of a flexible membrane (26) located adjacent to the corresponding divider walls (22a, 22b, 22c, 22d);
a selector (56, 154/156/168) moveable to selectively occlude the flowpaths to vary the flowrate of the infusion pump by compressing the corresponding valve seats; and
a motor (40) operably engaged to rotate the rotor to cause the first and second channel forming surfaces to pump a flow of fluid, the radial offsetting of the second channel forming surfaces from the first channel forming surfaces dampening a pulsatility of the flow of fluid.

2. The infusion pump (10, 110, 310, 410, 510, 610, 710, 810, 910a, 910b) according to claim 1, wherein the first channel forming surfaces (36a, 136a, 236a, 336, 436, 536, 636, 736, 836) are sized to produce a first flowrate, and the second channel forming surfaces (36b, 136b, 236b, 336, 436, 536, 636, 736, 836) are sized to produce a second flowrate different than the first flowrate.

3. The infusion pump (10, 110, 310, 410, 510, 610, 710, 810, 910a, 910b) according to claims 1 or 2, wherein the rotor (30, 130, 230, 330, 430, 530, 630, 730, 830) includes a third ring (38c, 138c) of surfaces (36c, 136c, 236c, 336, 436, 536, 636, 736, 836) that form channels with a third one of the plurality of flowpaths of the housing (12, 112, 312, 412, 512, 612a/612b, 712, 812), the third channel forming surfaces sized to produce a third flowrate different than the first and second flowrates, preferably wherein the third channel forming surfaces radially offset from the first and second channel forming surfaces to further dampen the pulsatility of the flow of fluid.

4. The infusion pump (10, 110, 310, 410, 510, 610, 710, 810, 910a, 910b) according to any one of the preceding claims, wherein the selector (56, 154/156/168) is configured to slide linearly to selectively (i) occlude flow to both the first ring (38a, 138a) of surfaces (36a, 136a, 236a, 336, 436, 536, 636, 736, 836) and the second ring (38b, 138b) of surfaces (36b, 136b, 236b, 336, 436, 536, 636, 736, 836), (ii) occlude flow to the second ring of surfaces only, or (iii) allow flow to both the first ring of surfaces and the second ring of channel forming surfaces, and/or
wherein the selector (56, 154/156/168) is configured to slide/linearly to selectively (i) close a first membrane valve (24i) for the first ring (38a, 138a) of channel forming surfaces (36a, 136a, 236a, 336, 436, 536, 636, 736, 836) and a second membrane valve (24a) for the second ring of channel forming surfaces, (ii) close the second membrane valve for the second ring (38b, 138b) of channel forming surfaces (36b, 136b, 236b, 336, 436, 536, 636, 736, 836) only, or (iii) allow both the first and second valves to be open and/or
wherein the selector (56, 154/156/168) is configured to rotate to selectively (i) occlude flow to both the first ring (38a, 138a) of channel forming surfaces (36a, 136a, 236a, 336, 436, 536, 636, 736, 836) and the second ring (38b, 138b) of channel forming surfaces (36b, 136b, 236b, 336, 436, 536, 636, 736, 836), (ii) occlude flow to the second ring of channel forming surfaces only, or (iii) allow flow to both the first ring of channel forming surfaces and the second ring of channel forming surfaces.

5. The infusion pump (10, 110, 310, 410, 510, 610, 710, 810, 910a, 910b) according to any one of the preceding claims, wherein the selector (56, 154/156/168) is configured to rotate to selectively (i) close a first tube (114a/116a) for the first ring (38a, 138a) of channel forming surfaces (36a, 136a, 236a, 336, 436, 536, 636, 736, 836) and a second tube (114b/116b) for the second ring (38b, 138b) of channel forming surfaces (36b, 136b, 236b, 336, 436, 536, 636, 736, 836), (ii) close the second tube for the second ring of channel forming surfaces only, or (iii) allow both the first and second tubes to be open, and/or
wherein the selector (56, 154/156/168) is configured to rotate to selectively (i) close a first tube (114a, 116a) for both the first ring (38a, 138a) of channel forming surfaces (36a, 136a, 236a, 336, 436, 536, 636, 736, 836) and the second ring (38b, 138b) of channel forming surfaces (36b, 136b, 236b, 336, 436, 536, 636, 736, 836), (ii) close a second tube (114b, 116b) for the second ring of channel forming surfaces only, or (iii) allow both the first and second tubes to be open, and/or
wherein the selector (56, 154/156/168) is configured to be rotated to selectively (i) close a first membrane valve (24i) for the first ring (38a, 138a) of channel forming surfaces (36a, 136a, 236a, 336, 436, 536, 636, 736, 836) and a second membrane valve (24a) for the second ring (38b, 138b) of channel forming surfaces (36b, 136b, 236b, 336, 436, 536, 636, 736, 836), (ii) close the second membrane valve, for the second ring of channel forming surfaces only, or (iii) allow both the first and second valves to be open.

6. The infusion pump (10, 110, 310, 410, 510, 610, 710, 810, 910a, 910b) according to any one of the preceding claims, wherein the housing (12, 112, 312, 412, 512, 612a/612b, 712, 812) and rotor (30, 130, 230, 330, 430, 530, 630, 730, 830) are part of a disposable portion of the infusion pump and/or wherein at least one of the motor (40) and the housing (12, 112, 312, 412, 512, 612a/612b, 712, 812) is part of a reusable portion of the infusion pump.

7. The infusion pump (10, 110, 310, 410, 510, 610, 710, 810, 910a, 910b) according to any one of the preceding claims, wherein the housing (12, 112, 312, 412, 512, 612a/612b, 712, 812) is connected to at least one of: a supply bag line, a patient line 916 and a supply bag (740, 840, 940).

8. The infusion pump (10, 110, 310, 410, 510, 610, 710, 810, 910a, 910b) according to any one of the preceding claims, wherein the housing (12, 112, 312, 412, 512, 612a/612b, 712, 812) and the rotor (30, 130, 230, 330, 430, 530, 630, 730, 830) are part of a first portion of the infusion pump and the selector (56, 154/156/168) and the motor (40) are part of a second portion of the infusion pump, the first and second portions being configured to be connected such that (i) the housing comes into operational communication with the selector and (ii) the rotor comes into operational communication with the motor, preferably wherein the first and second portions are configured to be connected such that that (i) the housing (12, 112, 312, 412, 512, 612a/612b, 712, 812) coming into operational communication with the selector (56, 154/156/168) and (ii) the rotor (30, 130, 230, 330, 430, 530, 630, 730, 830) coming into operational communication with the motor (40) occur virtually simultaneously.

9. The infusion pump (10, 110, 310, 410, 510, 610, 710, 810, 910a, 910b) according to claim 8, wherein the first and second portions are configured to be connected such that that (i) the housing (12, 112, 312, 412, 512, 612a/612b, 712, 812) coming into operational communication with the selector (56, 154/156/168) and (ii) the rotor (30, 130, 230, 330, 430, 530, 630, 730, 830) coming into operational communication with the motor (40) occur along a single axis of movement.

10. The infusion pump (10, 110, 310, 410, 510, 610, 710, 810, 910a, 910b) according to any one of the preceding claims, which is configured such that the housing (i) in a first position is operable to gravity feed a drug to a patient and (ii) in a second position is operable with the rotor (30, 130, 230, 330, 430, 530, 630, 730, 830) and the motor (40) to pump the drug to the patient.

11. The infusion pump (10, 110, 310, 410, 510, 610, 710, 810, 910a, 910b) according to claim 10, wherein the second position is obtained rotationally from the first position.

12. The infusion pump (10, 110, 310, 410, 510, 610, 710, 810, 910a, 910b) according to claim 10, wherein the second position is obtained translationally from the first position.

13. The infusion pump (10, 110, 310, 410, 510, 610, 710, 810, 910a, 910b) according to claim 10, which is provided with the housing (12, 112, 312, 412, 512, 612a/612b, 712, 812) initially in the first position.

14. The infusion pump (10, 110, 310, 410, 510, 610, 710, 810, 910a, 910b) according to any one of the preceding claims, which is coupled directly to a solution bag (740, 840, 940).

15. The infusion pump (10, 110, 310, 410, 510, 610, 710, 810, 910a, 910b) according to claim 14, which is configured such that the housing (i) in a first position is operable to gravity feed a drug to a patient and (ii) in a second position is operable with the rotor (30, 130, 230, 330, 430, 530, 630, 730, 830) and the motor (40) to pump the drug to the patient.

## Patentansprüche

1. Infusionspumpe (10, 110, 310, 410, 510, 610, 710, 810, 910a, 910b), die umfasst:
ein Gehäuse (12, 112, 312, 412, 512, 612a/612b, 712, 812), das (i) eine Vielzahl von Strömungswegen (20a, 20b, 20c, 20d, 20e), die durch jeweilige Trennwände (22a, 22b, 22c, 22d) getrennt sind, (ii) einen Einlass (14) und (iii) einen Auslass (16) definiert, wobei jeder der Vielzahl von Strömungswegen (20a, 20b, 20c, 20d, 20e) in einer Fluidverbindung mit dem Einlass (14) und dem Auslass (16) steht;
einen Rotor (30, 130, 230, 330, 430, 530, 630, 730, 830), der in das Gehäuse eingesetzt ist, wobei der Rotor einen ersten Ring (38a, 138a) aus Oberflächen (36a, 136a, 236a, 336, 436, 536, 636, 736, 836), die Kanäle mit einem ersten der Vielzahl von Strömungswegen des Gehäuses bilden, und einen zweiten Ring (38b, 138b) aus Oberflächen (36b, 136b, 236b, 336, 436, 536, 636, 736, 836) umfasst, die Kanäle mit einem zweiten der Vielzahl von Strömungswegen des Gehäuses bilden, wobei der zweite Kanal Oberflächen bildet, die radial gegen den ersten Kanal, der Oberflächen bildet, versetzt sind;
eine Vielzahl von Ventilsitzen bzw. Auflagern (24a, 24b, 24c, 24d, 24i), die Bereiche einer flexiblen Membran (26) umfassen, die benachbart zu den entsprechenden Trennwänden (22a, 22b, 22c, 22d) angeordnet sind;
eine Auswahleinrichtung (56, 154/156/168), die beweglich ist, um die Strömungswege selektiv zu verschließen, um den Durchsatz der Infusionspumpe durch Komprimieren der entsprechenden Ventilauflager zu variieren; und
einen Motor (40), der betriebsfähig in Eingriff ist, um den Rotor zu drehen, um zu bewirken, dass die ersten und zweiten kanalbildenden Oberflächen eine Fluidströmung pumpen, wobei der radiale Versatz der zweiten kanalbildenden Oberflächen gegenüber den ersten kanalbildenden Oberflächen eine Pulsatilität der Fluidströmung dämpft.

2. Infusionspumpe (10, 110, 310, 410, 510, 610, 710, 810, 910a, 910b) nach Anspruch 1, wobei die ersten kanalbildenden Oberflächen (36a, 136a, 236a, 336, 436, 536, 636, 736, 836) derart dimensioniert sind, dass sie einen ersten Durchsatz erzeugen, und die zweiten kanalbildenden Oberflächen (36b, 136b, 236b, 336, 436, 536, 636, 736, 836) derart dimensioniert sind, dass sie einen zweiten Durchsatz erzeugen, der sich von dem ersten Durchsatz unterscheidet.

3. Infusionspumpe (10, 110, 310, 410, 510, 610, 710, 810, 910a, 910b) nach Anspruch 1 oder 2, wobei der Rotor (30, 130, 230, 330, 430, 530, 630, 730, 830) einen dritten Ring (38c, 138c) aus Oberflächen (36c, 136c, 236c, 336, 436, 536, 636, 736, 836) umfasst, die Kanäle mit einem der dritten Vielzahl von Strömungswegen des Gehäuses (12, 112, 312, 412, 512, 612a/612b, 712, 812) ausbilden, wobei die dritten kanalbildenden Oberflächen derart dimensioniert sind, dass sie einen dritten Durchsatz erzeugen, der sich von den ersten und zweiten Durchsätzen unterscheidet, wobei die dritten kanalbildenden Oberflächen vorzugsweise radial gegen die ersten und zweiten kanalbildenden Oberflächen versetzt sind, um die Pulsatilität der Fluidströmung weiter zu dämpfen.

4. Infusionspumpe (10, 110, 310, 410, 510, 610, 710, 810, 910a, 910b) nach einem der vorhergehenden Ansprüche, wobei die Auswahleinrichtung (56, 154/156/168) aufgebaut ist, um linear zu gleiten, um selektiv (i) die Strömung sowohl zu dem ersten Ring (38a, 138a) aus Oberflächen (36a, 136a, 236a, 336, 436, 536, 636, 736, 836) und dem zweiten Ring (38b, 138b) aus Oberflächen (36b, 136b, 236b, 336, 436, 536, 636, 736, 836) zu verschließen, (ii) nur die Strömung zu dem zweiten Ring aus Oberflächen zu verschließen oder (iii) die Strömung sowohl zu dem ersten Ring aus Oberflächen als auch dem zweiten Ring aus kanalbildenden Oberflächen zuzulassen und/oder
wobei die Auswahleinrichtung (56, 154/156/168) konfiguriert ist, um linear zu gleiten, um selektiv (i) ein erstes Membranventil (24i) für den ersten Ring (38a, 138a) aus kanalbildenden Oberflächen (36a, 136a, 236a, 336, 436, 536, 636, 736, 836) und ein zweites Membranventil (24a) für den zweiten Ring aus kanalbildenden Oberflächen zu schließen, (ii) nur das zweite Membranventil für den zweiten Ring (38b, 138b) aus kanalbildenden Oberflächen (36b, 136b, 236b, 336, 436, 536, 636, 736, 836) zu schließen, oder (iii) zuzulassen, dass sowohl das erste als auch das zweite Membranventil offen sind, und/oder
wobei die Auswahleinrichtung (56, 154/156/168) konfiguriert ist, um sich zu drehen, um selektiv (i) die Strömung sowohl zu dem ersten Ring (38a, 138a) aus kanalbildenden Oberflächen (36a, 136a, 236a, 336, 436, 536, 636, 736, 836) als auch dem zweiten Ring (38b, 138b) aus kanalbildenden Oberflächen (36b, 136b, 236b, 336, 436, 536, 636, 736, 836) zu verschließen, (ii) nur die Strömung zu dem zweiten Ring aus kanalbildenden Oberflächen zu verschließen, oder (iii) die Strömung sowohl zu dem ersten Ring aus kanalbildenden Oberflächen als auch dem zweiten Ring aus kanalbildenden Oberflächen zuzulassen.

5. Infusionspumpe (10, 110, 310, 410, 510, 610, 710, 810, 910a, 910b) nach einem der vorhergehenden Ansprüche, wobei die Auswahleinrichtung (56, 154/156/168) konfiguriert ist, um sich zu drehen, um selektiv (i) ein erstes Rohr (114a/116a) für den ersten Ring (38a, 138a) aus kanalbildenden Oberflächen (36a, 136a, 236a, 336, 436, 536, 636, 736, 836) und ein zweites Rohr (114b/116b) für den zweiten Ring (38b, 138b) aus kanalbildenden Oberflächen (36b, 136b, 236b, 336, 436, 536, 636, 736, 836) zu schließen, (ii) nur das zweite Rohr für den zweiten Ring aus kanalbildenden Oberflächen zu schließen, oder (iii) zuzulassen, dass sowohl das erste als auch das zweite Rohr offen sind, und/oder
wobei die Auswahleinrichtung (56, 154/156/168) konfiguriert ist, um sich zu drehen, um selektiv (i) ein erstes Rohr (114a, 116a) sowohl für den ersten Ring (38a, 138a) aus kanalbildenden Oberflächen (36a, 136a, 236a, 336, 436, 536, 636, 736, 836) als auch den zweiten Ring (38b, 138b) aus kanalbildenden Oberflächen (36b, 136b, 236b, 336, 436, 536, 636, 736, 836) zu schließen, (ii) nur ein zweites Rohr (114b, 116b) für den zweiten Ring aus kanalbildenden Oberflächen zu schließen, oder (iii) zuzulassen, dass sowohl das erste als auch das zweite Rohr offen sind, und/oder
wobei die Auswahleinrichtung (56, 154/156/168) konfiguriert ist, um gedreht zu werden, um selektiv (i) ein erstes Membranventil (24i) für den ersten Ring (38a, 138a) aus kanalbildenden Oberflächen (36a, 136a, 236a, 336, 436, 536, 636, 736, 836) und ein zweites Membranventil (24a) für den zweiten Ring (38b, 138b) aus kanalbildenden Oberflächen (36b, 136b, 236b, 336, 436, 536, 636, 736, 836) zu schließen, (ii) nur das zweite Membranventil für den zweiten Ring aus kanalbildenden Oberflächen zu schließen, oder (iii) zuzulassen, dass sowohl das erste als auch zweite Membranventil offen ist.

6. Infusionspumpe (10, 110, 310, 410, 510, 610, 710, 810, 910a, 910b) nach einem der vorhergehenden Ansprüche, wobei das Gehäuse (12, 112, 312, 412, 512, 612a/612b, 712, 812) und ein Rotor (30, 130, 230, 330, 430, 530, 630, 730, 830) Teil eines Wegwerfabschnitts der Infusionspumpe sind und/oder wobei der Motor (40) und/oder das Gehäuse (12, 112, 312, 412, 512, 612a/612b, 712, 812) Teil eines wiederverwendbaren Abschnitts der Infusionspumpe ist/sind.

7. Infusionspumpe (10, 110, 310, 410, 510, 610, 710, 810, 910a, 910b) nach einem der vorhergehenden Ansprüche, wobei das Gehäuse (12, 112, 312, 412, 512, 612a/612b, 712, 812) mit einer Vorratsbeutelleitung und/oder einer Patientenleitung (916) und/oder einem Vorratsbeutel (740, 840, 940) verbunden ist.

8. Infusionspumpe (10, 110, 310, 410, 510, 610, 710, 810, 910a, 910b) nach einem der vorhergehenden Ansprüche, wobei das Gehäuse (12, 112, 312, 412, 512, 612a/612b, 712, 812) und der Rotor (30, 130, 230, 330, 430, 530, 630, 730, 830) Teil eines ersten Abschnitts der Infusionspumpe sind und die Auswahleinrichtung (56, 154/156/168) und der Motor (40) Teil eines zweiten Abschnitts der Infusionspumpe sind, wobei der erste und zweite Abschnitt derart aufgebaut sind, dass sie derart angeschlossen werden, dass (i) das Gehäuse in eine betriebsfähige Verbindung mit der Auswahleinrichtung kommt, und (ii) der Rotor in eine betriebsfähige Verbindung mit dem Motor kommt, wobei die ersten und zweiten Abschnitte vorzugsweise derart aufgebaut sind, dass sie derart angeschlossen werden, dass praktisch gleichzeitig stattfindet, dass (i) das Gehäuse (12, 112, 312, 412, 512, 612a/612b, 712, 812) in eine betriebsfähige Verbindung mit der Auswahleinrichtung (56/156/168) kommt, und (ii) der Rotor (30, 130, 230, 330, 430, 530, 630, 730, 830) in eine betriebsfähige Verbindung mit dem Motor (40) kommt.

9. Infusionspumpe (10, 110, 310, 410, 510, 610, 710, 810, 910a, 910b) nach Anspruch 8, wobei der erste und zweite Abschnitt derart aufgebaut sind, dass (i) das Gehäuse (12, 112, 312, 412, 512, 612a/612b, 712, 812) in eine betriebsfähige Verbindung mit der Auswahleinrichtung (56, 154/156/168) kommt, und (ii) der Rotor (30, 130, 230, 330, 430, 530, 630, 730, 830) in eine betriebsfähige Verbindung mit dem Motor (40) kommt, was entlang einer einzigen Bewegungsachse stattfindet.

10. Infusionspumpe (10, 110, 310, 410, 510, 610, 710, 810, 910a, 910b) nach einem der vorhergehenden Ansprüche, die derart konfiguriert ist, dass das Gehäuse (i) in einer ersten Position betriebsfähig ist, um ein Medikament durch die Schwerkraft an einen Patienten zuzuführen, und (ii) in einer zweiten Position mit dem Rotor (30, 130, 230, 330, 430, 530, 630, 730, 830) und dem Motor (40) betriebsfähig ist, um das Medikament zu dem Patienten zu pumpen.

11. Infusionspumpe (10, 110, 310, 410, 510, 610, 710, 810, 910a, 910b) nach Anspruch 10, wobei die zweite Position drehend aus der ersten Position erreicht wird.

12. Infusionspumpe (10, 110, 310, 410, 510, 610, 710, 810, 910a, 910b) nach Anspruch 10, wobei die zweite Position verschiebend aus der ersten Position erreicht wird.

13. Infusionspumpe (10, 110, 310, 410, 510, 610, 710, 810, 910a, 910b) nach Anspruch 10, die mit dem Gehäuse (12, 112, 312, 412, 512, 612a/612b, 712, 812) anfänglich in der ersten Position versehen ist.

14. Infusionspumpe (10, 110, 310, 410, 510, 610, 710, 810, 910a, 910b) nach einem der vorhergehenden Ansprüche, die direkt mit einem Lösungsbeutel (740, 840, 940) verbunden ist.

15. Infusionspumpe (10, 110, 310, 410, 510, 610, 710, 810, 910a, 910b) nach Anspruch 14, die derart konfiguriert ist, dass das Gehäuse (i) in einer ersten Position betriebsfähig ist, um ein Medikament durch die Schwerkraft an einen Patienten zuzuführen, und (ii) in einer zweiten Position mit dem Rotor (30, 130, 230, 330, 430, 530, 630, 730, 830) und dem Motor (40) betriebsfähig ist, um das Medikament zu dem Patienten zu pumpen.

## Revendications

1. Pompe de perfusion (10, 110, 310, 410, 510, 610, 710, 810, 910a, 910b) comprenant :
un logement (12, 112, 312, 412, 512, 612a/612b, 712, 812) définissant (i) une pluralité de trajets d'écoulement (20a, 20b, 20c, 20d, 20e) séparés par des parois de séparation (22a, 22b, 22c, 22d) respectives, (ii) une entrée (14), et (iii) une sortie (16), chacun de la pluralité de trajets d'écoulement (20a, 20b, 20c, 20d, 20e) étant en communication fluidique avec l'entrée (14) et la sortie (16) ;
un rotor (30, 130, 230, 330, 430, 530, 630, 730, 830) inséré dans le logement, le rotor comprenant une première bague (38a, 138a) de surfaces (36a, 136a, 236a, 336, 436, 536, 636, 736, 836) qui forment des canaux avec un premier de la pluralité de trajets d'écoulement du logement, et une deuxième bague (38b, 138b) de surfaces (36b, 136b, 236b, 336, 436, 536, 636, 736, 836) qui forment des canaux avec un second de la pluralité de trajets d'écoulement du logement, les deuxièmes surfaces de formation de canal étant radialement décalées par rapport aux premières surfaces de formation de canal ;
une pluralité des sièges de vanne (24a, 24b, 24c, 24d, 24i) comprenant des zones d'une membrane flexible (26) situées de manière adjacente aux parois de séparation (22a, 22b, 22c, 22d) correspondantes ;
un sélecteur (56, 154/156/168) pouvant être déplacé afin d'obturer de manière sélective les trajets d'écoulement, de manière à modifier le débit de la pompe de perfusion en comprimant les sièges de vanne correspondants ; et
un moteur (40) couplé de manière opérationnelle afin de faire tourner le rotor de manière à provoquer le pompage par les premières et deuxièmes surfaces de formation de canal d'un débit de fluide, l'excentration radiale des deuxièmes surfaces de formation de canal par rapport aux premières surfaces de formation de canal amortissant le caractère pulsé de l'écoulement de fluide.

2. Pompe de perfusion (10, 110, 310, 410, 510, 610, 710, 810, 910a, 910b) selon la revendication 1, dans laquelle les premières surfaces de formation de canal (36a, 136a, 236a, 336, 436, 536, 636, 736, 836) sont dimensionnées de manière à produire un premier débit, et les deuxièmes surfaces de formation de canal (36b, 136b, 236b, 336, 436, 536, 636, 736, 836) sont dimensionnées de manière à produire un deuxième débit différent du premier débit.

3. Pompe de perfusion (10, 110, 310, 410, 510, 610, 710, 810, 910a, 910b) selon les revendications 1 ou 2, dans laquelle le rotor (30, 130, 230, 330, 430, 530, 630, 730, 830) comporte une troisième bague (38c, 138c) de surfaces (36c, 136c, 236c, 336, 436, 536, 636, 736, 836) de formation de canal avec un troisième de la pluralité de trajets d'écoulement du logement (12, 112, 312, 412, 512, 612a/612b, 712, 812), les troisièmes surfaces de formation de canal étant dimensionnées de manière à produire un troisième débit différent des premier et deuxième débits, de préférence, dans laquelle les troisièmes surfaces de formation de canal sont radialement excentrées par rapport aux premières et deuxièmes surfaces de formation de canal, de manière à amortir davantage le caractère pulsé de l'écoulement de fluide.

4. Pompe de perfusion (10, 110, 310, 410, 510, 610, 710, 810, 910a, 910b) selon l'une quelconque des revendications précédentes, dans laquelle le sélecteur (56, 154/156/168) est configuré afin de pouvoir glisser linéairement pour, de manière sélective : (i) bloquer l'écoulement à la fois vers la première bague (38a, 138a) de surfaces (36a, 136a, 236a, 336, 436, 536, 636, 736, 836) et la deuxième bague (38b, 138b) de surfaces (36b, 136b, 236b, 336, 436, 536, 636, 736, 836), (ii) bloquer uniquement l'écoulement vers la deuxième bague de surfaces, ou (iii) permettre l'écoulement à la fois vers la première bague de surfaces et la deuxième bague de surfaces de formation de canal, et/ou
dans laquelle le sélecteur (56, 154/156/168) est configuré afin de pouvoir glisser linéairement pour, de manière sélective :(i) fermer une première vanne à membrane (24i) pour la première bague (38a, 138a) de surfaces de formation de canal (36a, 136a, 236a, 336, 436, 536, 636, 736, 836) et une seconde vanne à membrane (24a) pour la deuxième bague de surfaces de formation de canal, (ii) fermer uniquement la seconde vanne à membrane pour la deuxième bague (38b, 138b) de surfaces de formation de canal (36b, 136b, 236b, 336, 436, 536, 636, 736, 836), ou (iii) permettre à la fois l'ouverture des première et seconde vannes et/ou
dans laquelle le sélecteur (56, 154/156/168) est configuré afin de pouvoir tourner pour, de manière sélective : (i) bloquer l'écoulement vers la première bague (38a, 138a) de surfaces de formation de canal (36a, 136a, 236a, 336, 436, 536, 636, 736, 836) et la deuxième bague (38b, 138b) de surfaces de formation de canal (36b, 136b, 236b, 336, 436, 536, 636, 736, 836), (ii) bloquer uniquement l'écoulement vers la deuxième bague de surfaces, ou (iii) permettre l'écoulement à la fois vers la première bague de surfaces de formation de canal et la deuxième bague de surfaces de formation de canal.

5. Pompe de perfusion (10, 110, 310, 410, 510, 610, 710, 810, 910a, 910b) selon l'une quelconque des revendications précédentes, dans laquelle le sélecteur (56, 154/156/168) est configuré afin de pouvoir tourner pour, de manière sélective : (i) fermer un premier tube (114a/116a) pour la première bague (38a, 138a) de surfaces de formation de canal (36a, 136a, 236a, 336, 436, 536, 636, 736, 836) et un second tube (114b/116b) pour la deuxième bague (38b, 138b) de surfaces de formation de canal (36b, 136b, 236b, 336, 436, 536, 636, 736, 836), (ii) fermer uniquement le second tube pour la deuxième bague de surfaces de formation de canal ou (iii) permettre à la fois l'ouverture des premier et second tubes, et/ou
dans laquelle le sélecteur (56, 154/156/168) est configuré afin de pouvoir tourner pour, de manière sélective : (i) fermer un premier tube (114a, 116a) pour à la fois la première bague (38a, 138a) de surfaces de formation de canal (36a, 136a, 236a, 336, 436, 536, 636, 736, 836) et la deuxième bague (38b, 138b) de surfaces de formation de canal (36b, 136b, 236b, 336, 436, 536, 636, 736, 836), (ii) fermer uniquement un second tube (114b, 116b) pour la deuxième bague de surfaces de formation de canal, ou (iii) permettre à la fois l'ouverture des premier et second tubes, et/ou
dans laquelle le sélecteur (56, 154/156/168) est configuré afin de pouvoir tourner pour, de manière sélective : (i) fermer une première vanne à membrane (24i) pour la première bague (38a, 138a) de surfaces de formation de canal (36a, 136a, 236a, 336, 436, 536, 636, 736, 836) et une seconde vanne à membrane (24a) pour la deuxième bague (38b, 138b) de surfaces de formation de canal (36b, 136b, 236b, 336, 436, 536, 636, 736, 836), (ii) fermer uniquement la seconde vanne à membrane pour la deuxième bague de surfaces de formation de canal, ou (iii) permettre à la fois l'ouverture des première et seconde vannes.

6. Pompe de perfusion (10, 110, 310, 410, 510, 610, 710, 810, 910a, 910b) selon l'une quelconque des revendications précédentes, dans laquelle le logement (12, 112, 312, 412, 512, 612a/612b, 712, 812) et le rotor (30, 130, 230, 330, 430, 530, 630, 730, 830) sont des composants d'une partie jetable de la pompe de perfusion et/ou dans laquelle au moins l'un du moteur (40) et du logement (12, 112, 312, 412, 512, 612a/612b, 712, 812) est un composant d'une partie réutilisable de la pompe de perfusion.

7. Pompe de perfusion (10, 110, 310, 410, 510, 610, 710, 810, 910a, 910b) selon l'une quelconque des revendications précédentes, dans laquelle le logement (12, 112, 312, 412, 512, 612a/612b, 712, 812) est raccordé à au moins l'un parmi : une ligne de poche d'alimentation, une ligne de patient (916) et une poche d'alimentation (740, 840, 940).

8. Pompe de perfusion (10, 110, 310, 410, 510, 610, 710, 810, 910a, 910b) selon l'une quelconque des revendications précédentes, dans laquelle le logement (12, 112, 312, 412, 512, 612a/612b, 712, 812) et le rotor (30, 130, 230, 330, 430, 530, 630, 730, 830) sont des composants d'une première partie de la pompe de perfusion, et le sélecteur (56, 154/156/168) et le moteur (40) sont des composants d'une deuxième partie de la pompe de perfusion, les première et deuxième parties étant configurées de manière à être raccordées de telle sorte que (i) le logement entre en communication opérationnelle avec le sélecteur et (ii) le rotor entre en communication opérationnelle avec le moteur, de préférence, dans laquelle les première et deuxième parties sont configurées de manière à être raccordées de telle sorte que (i) l'entrée en communication opérationnelle du logement (12, 112, 312, 412, 512, 612a/612b, 712, 812) avec le sélecteur (56, 154/156/168) et (ii) l'entrée en communication opérationnelle du rotor (30, 130, 230, 330, 430, 530, 630, 730, 830) avec le moteur (40) se produisent théoriquement de manière simultanée.

9. Pompe de perfusion (10, 110, 310, 410, 510, 610, 710, 810, 910a, 910b) selon la revendication 8, dans laquelle les première et deuxième parties sont configurées de manière à être raccordées de telle sorte que (i) l'entrée en communication opérationnelle du logement (12, 112, 312, 412, 512, 612a/612b, 712, 812) avec le sélecteur (56, 154/156/168) et (ii) l'entrée en communication opérationnelle du rotor (30, 130, 230, 330, 430, 530, 630, 730, 830) avec le moteur (40) se produisent suivant un seul axe de mouvement.

10. Pompe de perfusion (10, 110, 310, 410, 510, 610, 710, 810, 910a, 910b) selon l'une quelconque des revendications précédentes, qui est configurée de telle sorte que le logement (i) dans une première position, peut être utilisé de manière à délivrer par gravité un médicament à un patient et (ii) dans une deuxième position, peut être utilisé avec le rotor (30, 130, 230, 330, 430, 530, 630, 730, 830) et le moteur (40) de manière à pomper le médicament vers le patient.

11. Pompe de perfusion (10, 110, 310, 410, 510, 610, 710, 810, 910a, 910b) selon la revendication 10, dans laquelle la deuxième position est obtenue par rotation à partir de la première position.

12. Pompe de perfusion (10, 110, 310, 410, 510, 610, 710, 810, 910a, 910b) selon la revendication 10, dans laquelle la deuxième position est obtenue par translation à partir de la première position.

13. Pompe de perfusion (10, 110, 310, 410, 510, 610, 710, 810, 910a, 910b) selon la revendication 10, qui est équipée du logement (12, 112, 312, 412, 512, 612a/612b, 712, 812) initialement dans la première position.

14. Pompe de perfusion (10, 110, 310, 410, 510, 610, 710, 810, 910a, 910b) selon l'une quelconque des revendications précédentes, qui est couplée directement à une poche de solution (740, 840, 940).

15. Pompe de perfusion (10, 110, 310, 410, 510, 610, 710, 810, 910a, 910b) selon la revendication 14, qui est configurée de telle sorte que le logement (i) dans une première position, peut être utilisé de manière à délivrer par gravité un médicament à un patient et (ii) dans une deuxième position peut être utilisé avec le rotor (30, 130, 230, 330, 430, 530, 630, 730, 830) et le moteur (40) de manière à pomper le médicament vers le patient.
